(19) Europäisches Patentamt
European Patent Office
Office européen des brevets

(11) Veröffentlichungsnummer: **0 000 330**
**B1**

(12) **EUROPÄISCHE PATENTSCHRIFT**

(45) Veröffentlichungstag der Patentschrift: **05.08.81**

(21) Anmeldenummer: **78100149.0**

(22) Anmeldetag: **14.06.78**

(51) Int. Cl.³: **C 07 C 103/52, A 61 K 37/02**

# (54) Lipopeptide, Verfahren zu deren Herstellung und sie enthaltende pharmazeutische Präparate.

(30) Priorität: **20.06.77 LU 77584**

(43) Veröffentlichungstag der Anmeldung: **24.01.79 Patentblatt 79/2**

(45) Bekanntmachung des Hinweises auf die Patenterteilung: **05.08.81 Patentblatt 81/31**

(84) Benannte Vertragsstaaten: **BE CH DE FR GB LU NL SE**

(56) Entgegenhaltungen:
**NL - A - 72 06136**
**NL - A - 73 12987**

(73) Patentinhaber: **CIBA-GEIGY AG**
**Patentabteilung Postfach**
**CH-4002 Basel (CH)**

(72) Erfinder: **Tarcsay, Lajos Zoltan, Dr.**
**Muttenzerstrasse 25**
**D-7889 Grenzach-Wyhlen (DE)**
Erfinder: **Kamber, Bruno, Dr.**
**Sonnenweg 9**
**CH-4144 Arlesheim (CH)**
Erfinder: **Stanek, Jaroslav, Dr.**
**Florastrasse 6**
**CH-4127 Birsfelden (CH)**
Erfinder: **Baschang, Gerhard, Dr.**
**Bückenweg 7**
**CH-4126 Bettingen (CH)**
Erfinder: **Hartmann, Albert, Dr.**
**Steingasse 21A**
**D-7889 Grenzach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 330 B1

# Lipopeptide, Verfahren zu deren Herstellung und sie enthaltende pharmazeutische Präparate

Die Erfindung betrifft neue Lipopeptide der Formel

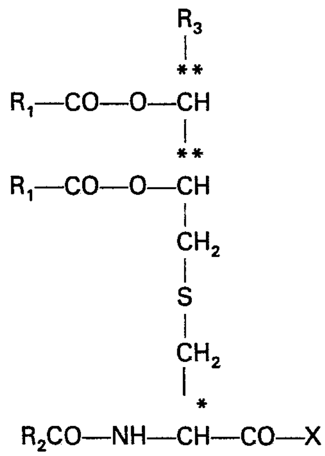

(I)

* = R

** = R oder S

worin $R_1$ und $R_2$ je einen gesättigten oder ungesättigten, aliphatischen oder gemischt aliphatischcycloaliphatischen, gegebenenfalls auch durch Sauerstoffunktionen substituierten, Kohlenwasserstoffrest mit 11—21 C-Atomen, $R_3$ Wasserstoff oder den Rest $R_1$—CO—O—$CH_2$— wo $R_1$ die gleiche Bedeutung hat, und X eine peptidisch gebundene natürliche aliphatische Aminosäure mit freier, veresterter oder amidierter Carboxylgruppe, oder eine Aminosäurensequenz von 2—10 natürlichen aliphatischen Aminosäuren, deren terminale Carboxylgruppe in freier, veresterter oder amidierter Form vorliegt, bedeuten, wobei die mit * bzw. ** bezeichneten Asymmetrie-Zentren die absolute R- bzw. S- oder R-Konfiguration besitzen, und gegebenenfalls Gemische der an den C**-Atomen epimeren R- und S-Verbindungen, und gegebenenfalls Salze und Komplexe dieser Verbindungen, sowie Verfahren zu ihrer synthetischen Herstellung, und pharmazeutische Präparate enthaltend einen oder mehrere dieser Lipopeptide zusammen mit einem pharmazeutischen Trägermaterial und gegebenenfalls zusammen mit anderen pharmazeutischen Verbindungen.

Lipopeptide sind als Abbau-Fragmente von Lipoproteinen bereits beschrieben worden. So haben z.B. Hantke und Braun (Eur. J. Biochem. *34*, 284—296 (1973) aus dem Murein-Lipoprotein der äusseren Zellwand von Escherichia coli durch enzymatische Degradation Lipopeptide bzw. Lipopeptidgemische in unreiner Form isolieren können, denen gemäss ihren Untersuchungen die folgende Struktur

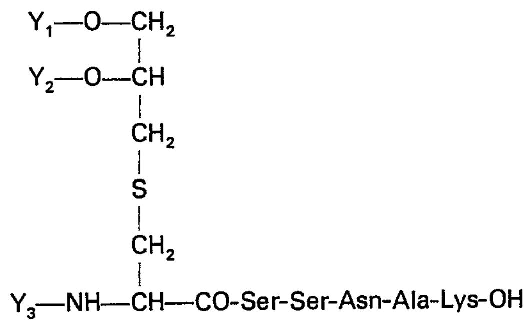

(II)

oder entsprechende Formeln mit verkürzter Polypeptid-Kette zukommen dürfte, wobei $Y_1$, $Y_2$, $Y_3$ Acylreste verschiedener höherer gesättigter und ungesättigter Fettsäuren, wie etwa solcher mit 14—19 C-Atomen und anderer, darstellen. Dieselbe Struktur liegt auch dem Murein-Protein als solchem zugrunde (loc.cit.), wobei die absolute Konfiguration an den beiden asymmetrischen Kohlenstoffatomen der obigen Formel nicht untersucht wurde. Sowohl das Murein-Lipoprotein wie seine genannten Abbauprodukte zeigen in vitro eine mitogene Wirkung gegenüber Mäuse-Lymphocyten. [Vgl. auch Z. Immun.-Forschung, Vol. 153, pp. 11—22 (1977)].

Wie ersichtlich, liegt den aus Mureinprotein erhaltenen Abbau-Lipopeptiden das "Glyzerylcystein"

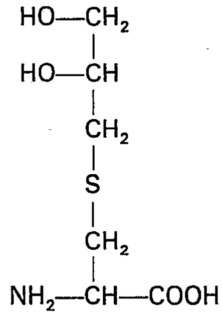

(III)

zugrunde. Als Produkte einer enzymatischen Spaltung von Naturprodukten waren diese Fragmente keine in ihrer Zusammensetzung wohl definierten Produkte und stellten offensichtlich komplizierte Gemische von Kondensationsprodukten aus dem genannten Peptidanteil und sehr verschiedenen Acylderivaten des Glyzerylcysteins dar.

Die Lipopeptide der vorliegenden Anmeldung unterscheiden sich nun von den genannten Abbauprodukten von Murein-Proteinen in mancherlei Hinsicht: sie stellen Lipopeptide von genau definierter einheitlicher chemischer Konstitution und Konfiguration dar, die synthetisch zugänglich sind und sich daher für eine therapeutische Anwendung eignen; die von ihnen gebildete Gruppe umfasst Verbindungen, in denen anstelle des Glyzerinanteils im "Glyzerylcystein" der Rest des Erythrits tritt und/oder solche, in denen eine andere Aminosäurensequenz als die oben für die bekannten Degradations-Lipopeptide-Gemische in Formel (II) angegebene, oder an deren Stelle auch nur eine einzige Aminosäure vorhanden ist, und schliesslich auch Derivate, wie Amide und Ester der terminalen Carboxylgruppe.

In den Acylresten $R_1CO$, $R_2CO$ in den Verbindungen der Formel I und ihren Derivaten sind $R_1$ und $R_2$ gesättigte oder ungesättigte, aliphatische oder aliphatisch-cycloaliphatische, gegebenenfalls auch durch Sauerstoffunktionen substituierte, Kohlenwasserstoffreste mit 11—21 C-Atomen, d.h. die Acylreste leiten sich von gesättigten oder ungesättigten, aliphatischen oder cycloaliphatischen, gegebenenfalls im Kohlenwasserstoffrest oxygenierten Carbonsäuren mit 12—22, vorzugsweise mit 14—18, C-Atomen ab. Als solche sind die gesättigten oder ungesättigten Fettsäuren zu nennen, wie Laurinsäure, Myristinsäure, Palmitinsäure, Margarinsäure, Stearinsäure, Arachinsäure, Oelsäure, Elaidinsäure, Linolsäure, $\alpha$- und $\beta$-Eläostearinsäure, Stearolsäure, $\alpha$-Linolensäure, ferner unter den cycloaliphatisch-aliphatischen z.B. die Dihydrosterkulsäure, Malvalsäure, Hydnocarpussäure, und Chaulmoograsäure. Oxygenierte Säuren dieses Typus, welche ebenfalls für die Acylreste $R_1CO$ und $R_2CO$ in Betracht kommen, sind z.B. die durch Epoxydierung der oben genannten olefinischen Fettsäuren und cycloaliphatisch-aliphatischen Säuren sich ergebenden, z.B. die $\delta,\iota$-Epoxystearinsäure, ferner Derivate der oben genannten Säuren, die z.B. eine oder mehrere Hydroxygruppen aufweisen, wie z.B. die Ricinolsäure.

In den Verbindungen der Formel (I) bzw. ihren Diastereomeren-Gemischen und ihren Salzen oder Komplexen können die Gruppen $R_1$ und $R_2$ miteinander identisch oder voneinander verschieden sein. Bevorzugt sind Verbindungen, in denen alle drei bzw. vier Acylreste gleich sind und unter diesen insbesondere solche, in denen diese den Palmitoyl-, Stearoyl- oder Oleoylrest bedeuten.

Die Peptidsequenz X in den Verbindungen der Formel (I) bzw. in deren Salzen setzt sich aus maximal 10 natürlichen aliphatischen Aminosäuren zusammen, wobei vorzugsweise mindestens die Hälfte eine hydrophile Gruppe tragen, wie insbesondere Hydroxy-, Amino, Carboxyl-, Carbamid-, Guanidino- oder Imidazolylgruppen. Von den ionischen Aminosäuren dieser Kategorie seien unter den saure Gruppen tragenden insbesondere die Asparagin- und Glutamin- und die Oxyglutaminsäure hervorgehoben, unter den basische Gruppen tragenden das Lysin, das Ornithin, das Arginin und das Histidin. Aminosäuren mit neutralem Charakter sind besonders die Amide, wie das Asparagin, das Glutamin und die Hydroxygruppen tragenden, in erster Linie Serin und Threonin.

Stellt X in Formel (I) die genannte Aminosäure dar, so ist sie ebenfalls z.B. eine der genannten hydrophile Gruppen tragenden, z.B. insbesondere Serin oder Threonin.

Unter den Aminosäuren, die in die obige Sequenz eingehen können und die keine hydrophilen Gruppen tragen, sind vor allem die unsubstituierten zu erwähnen, wie z.B. Glycin, Alanin, Valin, Norvalin, Leucin, Isoleucin, sodann aber auch einige substituierte, die nicht-hydrophilen Charakter haben, wie Methionin.

Die Reihenfolge der genannten Aminosäuren in der Peptidkette X kann beiliebig sein, jedoch sind solche Sequenzen bevorzugt, in denen sämtliche Aminosäuren mit hydrophilen Gruppen direkt aneinander gebunden sind, und unter diesen wiederum diejenigen, in welchen diese Sequenz der hydrophilen Aminosäuren an die Carboxylgruppe des Cysteins im Triacyl-Glyzerylcystein bzw. Tetraacyl-Erythritylcystein-Teil gebunden ist.

Eine wichtige Gruppe [Gruppe I] von Lipopeptiden gemäss der vorliegenden Erfindung sind solche, in denen die Peptidkette X aus maximal 5 Aminosäuren besteht. Unter diesen sind insbesondere solche der Formel

$$
\begin{array}{c}
R_1\text{—CO—O—}CH_2 \\
| \\
** \\
R_1\text{—CO—O—}CH \\
| \\
CH_2 \\
| \\
S \\
| \\
CH_2 \\
| \\
* \\
R_2CO\text{—NH—CH—CO—}X
\end{array}
\qquad (IV)
$$

$* = R$

$** = R$

worin $R_1$, $R_2$ die gleichen Bedeutungen besitzen und X die für Formel (I) gegebene Bedeutung hat, aber im Falle der Aminosäurensequenz 2—5 Aminosäuren aufweist, in welcher vorzugsweise mindestens die Hälfte eine hydrophile Gruppe tragen, sowie ihre Salze und Komplexe. Unter diesen Lipopeptiden seien jene hervorgehoben, deren Aminosäurensequenz in der Gruppe X derjenigen der obengenannten durch Abbau von Murein-Lipoprotein erhaltenen Lipopeptide d.h. solcher der Formel (II), oder einer sich davon ableitenden verkürzten Kette entspricht. Es seien insbesondere folgende Lipopeptid-Typen erwähnt.

(AcGCT = Acyl-Glyzerylcystein-Teil gemäss Formel IV):
AcGCT-Ser-Ser-Asn-Ala-Lys-OH
AcGCT-Ser-Ser-Asn-Ala-OH
AcGCT-Ser-Ser-Asn-OH
AcGCT-Ser-Ser-OH,

sodann die entsprechenden Typen, in welchen Threonin, Glutamin oder Asparagin das Serin ersetzen; ferner die Verbindungstypen der Art:

AcGCT-Ser-Ser-Asn-Ala-Glu-OH
AcGCT-Ser-Ser-Phe-Ala-Glu-OH
AcGCT-Ser-Ser-Phe-Ala-OH
AcGCT-Ser-Ser-Phe-OH

und die entsprechenden mit Threonin, Glutamin oder Asparagin als Austauschaminosäuren für Serin, und die Amide und Carbonsäureester mit terminaler Carbamid- bzw. Estergruppe, wobei in erster Linie die Verbindungen in Betracht zu ziehen sind, in denen die Acylreste $R_1$—CO, $R_2$—CO im Acyl-Glyzeryl-cystein-Teil gleich sind und Palmitoyl, Stearoyl oder Oleoyl darstellen, und alle diejenigen, in denen $R_1$—CO und $R_2$—CO verschieden sind und z.B. Palmitoyl, Stearoyl oder Oleoyl bedeuten, und diese Reste in beliebiger Kombination und/oder Variation vorkommen, wie die in der folgenden Tabelle angeführten, wobei der Glyzerylcystein-teil entsprechend den N- und O-Acylsubstituenten als N-Acyl-O—O-di-Acyl-Cys abgekürzt geschrieben ist:

N-Palmitoyl-O.O-di-Palmitoyl-Cys-Ser-Ser-Asn-Ala-Lys-OH
N-Palmitoyl-O.O-di-Palmitoyl-Cys-Ser-Ser-Asn-Ala-OH
N-Palmitoyl-O.O-di-Palmitoyl-Cys-Ser-Ser-Asn-OH
N-Palmitoyl-O.O-di-Palmitoyl-Cys-Ser-Ser-OH
N-Palmitoyl-O.O-di-Palmitoyl-Cys-Ser-OH
N-Stearoyl-O.O-di-Stearoyl-Cys-Ser-Ser-Asn-Ala-Lys-OH
N-Stearoyl-O.O-di-Stearoyl-Cys-Ser-Ser-Asn-Ala-OH
N-Stearoyl-O.O-di-Stearoyl-Cys-Ser-Ser-Asn-OH
N-Stearoyl-O.O-di-Stearoyl-Cys-Ser-Ser-OH
N-Stearoyl-O.O-di-Stearoyl-Cys-Ser-OH
N-Oleoyl-O.O-di-Oleoyl-Cys-Ser-Ser-Asn-Ala-Lys-OH
N-Oleoyl-O.O-di-Oleoyl-Cys-Ser-Ser-Asn-Ala-OH
N-Oleoyl-O.O-di-Oleoyl-Cys-Ser-Ser-Asn-OH

N-Oleoyl-O.O-di-Oleoyl-Cys-Ser-Ser-OH
N-Oleoyl-O.O-di-Oleoyl-Cys-Ser-OH
N-Lauroyl-O.O-di-Lauroyl-Cys-Ser-Ser-Asn-Ala-Lys-OH
N-Lauroyl-O.O-di-Lauroyl-Cys-Ser-Ser-Asn-Ala-OH
N-Lauroyl-O.O-di-Lauroyl-Cys-Ser-Ser-Asn-OH
N-Lauroyl-O.O-di-Lauroyl-Cys-Ser-Ser-OH
N-Lauroyl-O.O-di-Lauroyl-Cys-Ser-OH

ferner die Verbindungen, die sich aus der Substitution des Serins durch Threonin, Glutaminsäure, Glutamin, Asparaginsäure oder Asparagin, aus der Substitution des Lysins durch Glutaminsäure, und solche, die sich aus der gleichzeitigen Substitution von Lysin durch Glutaminsäure und Asparagin durch Phenylalanin ergeben.

Unter den Verbindungen mit verschiedenen Acylresten an N- und O-Atomen im glyzerylcystein-Rest, sind die folgenden hervorzugheben:

N-Myristoyl-O.O-di-Palmitoyl-Cys-Phe-Phe-Asn-Ala-Lys-OH
N-Lauroyl-O.O-di-Palmotoyl-Cys-Ser-Ser-Asn-Ala-Glu-OH
N-Stearoyl-O.O-di-Palmitoyl-Cys-Ser-Ser-Asn-Ala-Ala-OH

und die sich durch Ersatz von Ser durch Phe oder von Phe durch Ser ergebenden Verbindungen.

Eine zweite Gruppe von Verbindungen [Gruppe II] gemäss der Erfindung sind Verbindungen entsprechend der obigen Formel IV, in denen jedoch die Konfiguration am C**-Atom S statt R ist, und die Gemische der R- und S-Diastereomeren, wie sie z.B. bei der synthetischen Darstellung anfallen können. Spezifische Typen und Verbindungen entsprechen auch in dieser Gruppe den oben für die "R"-Gruppe (Formel IV) besonders hervorgehobenen.

Eine dritte Gruppe [Gruppe III] der neuen Lipopeptide umfasst die Verbindungen gemäss Formel (IV), in denen jedoch X eine Aminosäurensequenz mit 6—10 Aminosäuren darstellt, wobei die ersten 5 vorzugsweise die oben bereits hervorgehobenen Sequenzen darstellen. Die Aminosäuren 6—10 können beliebige der oben erwähnten natürlichen sein, wie insbesondere Serin, Asparagin, Alanin, Glutaminsäure, Lysin, Phenylalanin, und etwa in folgenden Sequenzen vorkommen:

Ile-Asp-Glu-OH
Asp-Glu-OH
Spezifische Verbindungen sind z.B.
N-Stearoyl-S-[2(R),3-Dipalmitoyloxypropyl]-Cys-Ser-Ser-Asn-Ala-Glu-Ile-Asp-Glu-OH
N-Palmitoyl-S-[2(R),3-Dipalmitoyloxypropyl]-Cys-Ser-Ser-Asn-Ala-Glu-Ile-Asp-Glu-OH
N-Palmitoyl-S-[2(R),3-dipalmitoyloxypropyl]-Cys-Ser-Ser-Asn-Ala-Lys-Ile-Asp-Glu-OH.

Es können erfindungsgemäss auch entsprechende Verbindungen dieses Typs mit der S-Konfiguration am C**-Atom hergestellt werden, sowie Diastereomerengemische von S- und R-Verbindungen.

Eine vierte Gruppe [Gruppe IV] der Lipopeptide gemäss der Erfindung umfasst Verbindungen der Formel (I) worin $R_3$ den Rest $R_1$—CO—O—$CH_2$— darstellt, in welchen die Konfiguration an den C**-Atomen eine beliebige ist, insbesondere auch Diastereomerengemische. Untergruppen sind Verbindungen der Formel

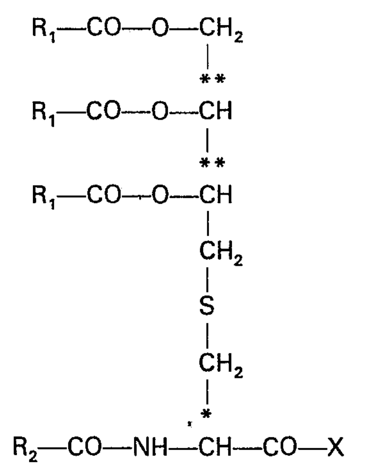

(V)

* = R

** = R oder S

insbesondere solche mit R-Konfiguration an den C**-Atomen, worin X die für Formel (IV) angegebene Bedeutung besitzt. Die Acylreste $R_1$—CO— und $R_2$—CO— sind vorzugsweise dieselben und vor allem wiederum Palmitoyl, Stearoyl oder Oleoyl; sie können aber wie oben für die anderen Gruppen besprochen. in allen Kombinationen und/oder Variationen vorhanden sein.

Bevorzugte Typen und Verbindungen dieser Klasse sind wiederum solche, in denen X die oben spezifizierten Bedeutungen haben.

Auch für diese Gruppe ist vorzugsweise X eine Aminosäure oder eine Sequenz von 2—5 Aminosäuren, insbesondere eine der oben für die anderen drei Gruppen von Lipopeptiden als bevorzugt bezeichneten. X kann aber auch eine der für die dritte Gruppe der Lipopeptide der vorliegenden Anmeldung beschriebenen Aminosäurensequenzen mit 6—10 Aminosäuren, insbesondere die für jene Gruppe spezifisch erwähnten, darstellen.

Spezifische Verbindungen sind z.B. die sich aus der obigen für spezifische Verbindungen der Gruppe I zusammengestellte Tabelle ergebenden, wenn in ihnen N-Acyl-O—O-di-Acyl-Cys nicht wie dort den Glyzerylcysteinanteil, sondern den Erythritylcystein-Rest bedeutet, und die sich durch Substitution des Serins durch Threonin, Glutaminsäure, Glutamin, Asparaginsäure oder Asparagin und von Lysin durch Glutaminsäure und, wenn erwünscht, gleichzeitig von Asn durch Phenylalanin ergebenden Verbindungen.

Eine fünfte Gruppe von Lipopeptiden gemäss der vorliegenden Erfindung wird von jenen Verbindungen gemäss Formel (I) gebildet, in denen X eine Peptidkette bedeutet, in welchen die Sequenz der ersten 5 Aminosäuren von denjenigen, die in den bekannten Murein — Lipoprotein — Abbau — Lipopeptiden vorhandenen verschieden ist, und insbesondere solche worin $R_3$ Wasserstoff bedeutet, also der obigen Formel (IV) entsprechen, wobei $R_1$—CO und $R_2$—CO die vorzugsweise im vorangegangenen oder im folgenden besonders hervorgehobenen Acylreste in den angeführten Kombinationen bedeuten, sowie ihre Salze und Komplexe. Es seien z.B die folgenden Lipopeptid — Typen erwähnt (AcGCT = Acyl-Glyzerylcystein-Teil gemäss Formel (IV)

AcGCT-Phe-Ile-Ile-Phe-Ala-OH
AcGCT-Val-Lys-Val-Tyr-Pro-OH

und ihre Amide und Ester, insbesondere die im nachfolgend besonders hervorgehobenen.

Eine weitere Untergruppe besteht aus Verbindungen gemäss Formel (IV), in denen X aber die besagte "unnatürliche" Sequenz von Aminosäuren im Vergleich zu den Murein-Lipoprotein-Degradationsprodukten darstellt. und aber 6—10 Aminosäuren vorhanden sind. Die ersten fünf Aminosäuren haben z.B. die oben angegebene Sequenz oder eine beliebige andere; es seien z.B. die folgenden Lipopeptide genannt, wo AcGCT die obige Bedeutung besitzt:

AcGCT-Ala-Ile-Gly-Val-Gly-Ala-PRo-OH
AcGCT-Ser-Ser-Asn-Ala-Glu-Ile-Asp-Glu-OH

und ihre Amide und Ester, insbesondere die im nachfolgenden hervorgehobenen.

Vorzugsweise werden Verbindungen dieses Typs hergestellt, in denen die Konfiguration am C**-Atom des AcGCT R ist, oder Gemische von R- und S-Epimeren.

In den oben besprochenen neuen Lipopeptiden gemäss der vorliegenden Erfindung kann die terminale Carboxylgruppe in amidierter Form vorliegen. Ausser dem unsubstituierten Amid kommen auch solche in Betracht, die sich von einem primären oder sekundären Amin ableiten. Insbesondere kommen Amide von niederen aliphatischen Aminen mit 1—7 C-Atomen in Betracht, wie Methylamin, Ethylamin, Propylamin, Butylamin, Dimethylamin, Diäthylamin oder Propyl- und Isopropylamin, ferner aromatische Amine, insbesondere monocyclische, wie Anilin oder Toluidin, araliphatische, wie Benzylamin oder heterocyclische Amine, wie z.B. die Aminopyridine. Bei den sekundären aliphatischen Aminen kann es sich auch um ringgeschlossene Stickstoffbasen handeln, wie z.B. Pyrrolidin, Piperidin oder Piperazin. Spezifische Amide sind z.B. die unsubstituierten oder die Methyl-, Aethyl-, Dimethyl- oder Diäthylamide sämtlicher oben angeführter spezifischer Lipopeptide gemäss der vorliegenden Erfindung oder solche Amide der in der illustrativen Beispielen beschriebenen Verbindungen.

In den veresterten terminalen Carboxylgruppen der neuen Lipopeptide leitet sich die Alkoholkomponente vorzugsweise von niederen aliphatischen Alkoholen mit 1—7 C-Atomen, wie Methyl-, Ethyl-, n-Propyl-, Isopropyl- oder den Butylalkoholen ab. Die veresternden Alkohole können aber auch mehrwertige sein, wie Aethylenglykol oder Propylenglykol oder Glyzerin. Auch araliphatische Alkohole, insbesondere monocyclische niederaliphatische mit 1—7 C-Atomen im aliphatischen Teil, wie z.B. Benzylalkohol, oder heterocyclische Alkohole, wie Tetrahydrofuranol oder Tetrahydropyranol können für die Veresterung verwendet werden. Als spezifische Ester dieses Typs der Lipopeptide gemäss der Erfindung seien z.B. die Methyl-, Aethyl- und die Aethylenglykol- oder Propylenglykol-Ester sämtlicher oben angeführter spezifischer Lipopeptide oder derjenigen, die in den illustrativen Beispielen beschrieben sind, erwähnt.

Die vorliegenden neuen Lipopeptide sind je nach der Art ihrer Substituenten neutrale, saure oder basische Verbindungen. Falls überschüssige saure Gruppen vorhanden sind, bilden sie Salze mit Basen,

wie Ammoniumsalze oder Salze mit Alkali- oder Erdalkalimetallen, z.B. Natrium, Kalium, Calcium oder Magnesium; sind jedoch überschüssige basische Gruppen vorhanden, bilden sie Säureadditionssalze.

Säureadditionssalze sind besonders pharmazeutisch verwendbare, nichttoxische Säureadditionssalze, wie solche mit anorganischen Säuren, z.B. Chlorwasserstoff-, Bromwasserstoff-, Salpeter-, Schwefel- oder Phosphorsäuren, oder mit organischen Säuren, wie organischen Carbonsäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Amino-salicylsäure, 2-Phenoxybenzoesäure, 2-Acetoxy-benzoesäure, Embonsäure, Nicotinsäure oder Isonicotinsäure, oder organischen Sulfonsäuren, z.B. Methansulfonsäure, Aethansulfonsäure, 2-Hydroxy-äthansulfonsäure, Aethan-1,2-disulfonsäure, Benzolsulfonsäure, p-Toluolsulfonsäure oder Naphthalin-2-sulfonsäure, ferner auch andere Säureadditionssalz, die z.B. als Zwischenprodukte, z.B. zur Reinigung der freien Verbindungen oder in der Herstellung von anderen Salzen, sowie zur Charakterisierung verwendet werden können, wie z.B. solche mit Pikrin-, Pikrolon-, Flavian-, Phosphorwolfram-, Phosphormolyddän-, Chlorplatin-, Reinecke- oder Perchlorsäure.

Komplexe sind die mit Metallsalzen, z.B. mit Schwermetallsalzen, wie Kupfer, Zink, Eisen oder Kobaltsalzen entstehenden Verbindungen. Zur Bildung solcher Komplexe werden vorzugsweise die Phosphate, Pyrophosphate und Polyphosphate dieser Metallsalze verwendet, gegebenenfalls in Kombination mit sauren organischen Stoffen, z.B. saure Gruppen enthaltenden Polysacchariden, wie Carboxymethylcellulose, Gerbsäure, Polyglutaminsäure oder teilweise hydrolysierte Gelatine, ferner Alkalimetallpolyphosphate wie z.B. "Calgon N", "Calgon 322", "Calgon 188" oder "Plyron B 12".

Die Verbindungen der vorliegenden Erfindung gemäss der obigen Formel (I), ihre Salze und Komplexe und Gemische weisen wertvolle pharmakologische Eigenschaften, insbesondere eine ausgeprägte immunpotenzierende Wirking auf. So stimulieren die Verbindungen im Dosisbereich von 0,5—320 μg/ml die in vitro an Hand der Thymidininkorporation bestimmte Proliferation von B-Lymphocyten 20- bis 50-fach im Vergleich zu nicht stimulierten Kontroll-Lymphocyten. Das Ausmass der Stimulation entspricht demjenigen, das mit den wirksamsten bekannten B-Zell-Mitogenen [Dextran-sulfat, E. coli Lipopolysaccharid, PPD (purified protein derivative)] erreicht wird, wobei bei den neuen Verbindungen der vorliegenden Anmeldung auch hohe Konzentrationen nicht lymphocytotoxisch wirken.

Die neuen Verbindungen der Formel (I), ihre Salze und Komplexe und Gemische sind ausserdem in der Lage, in Konzentrationen von 0,3—60 μg/ml in Milzzellkulturen von normalen Mäusen die Bildung antikörperproduzierender Zellen zu induzieren (Vermehrung der 19S-plaquebildenden Zellen um einen Faktor 20 bis 50 über den Kontrollwert (in Abwesenheit der stimulierenden Substanzen)): So werden in Anwesenheit der genannten Verbindungen z.B. spezifische Antikörper gegen Schaferythrocyten gebildet, ohne dass den Kulturen Schaferythrocyten zur Immunisierung zugesetzt werden. Andererseits vermögen die genannten Substanzen im selben Konzentrationsbereich auch die immunologische Reaktivität von T-zellverarmten Milzzellkulturen (von kongenital athymischen nu/nu Mäusen) gegenüber einem normalerweise thymusabhängigen Antigen (Schaferythrocyten) zu steigern (faktor 10 bis 40 gegenüber unbehandelten Kontrollkulturen). Durch die genannten Verbindungen werden aber in vitro direkt oder indirekt nicht nur Proliferations- und Syntheseleistungen von B-Lymphocyten (d. h. von potentiell antikörperbildenden Zellen) induziert, sondern auch Effekte auf T-Lymphocyten (zu denen regulatorisch aktive Helfer- und Suppressorzellen sowie cytotoxische Effektorzellen gehören), vermittelt. So vermögen z.B. die erwähnten Verbindungen in einem Konzentrationsbereich von 10—100 μg/ml die Reaktivität von cortisonresistenten Thymuszellen gegenüber allogenen bestrahlten Stimulatorlymphocyten erheblich (bis zu 10-fach) zu potenzieren.

Die oben erwähnten wirkungen kommen wahrscheinlich indirekt dadurch zustande, dass die Lipopeptide Makrophagen aktivieren, die ihrerseits die Reaktivität von T- und B-Lymphocyten fördern. Tatsächlich kann man zeigen, dass die genannten Verbindungen bereits in äusserst geringen Konzentrationen (0,01—10 μg/ml) grosse Mengen "colony stimulating activity" (CSA) aus Maus-Makrophagen freisetzen (Induktion von bis zu 150—200 Kolonien innert 7 Tagen aus $10^5$ Mäuse-Knochenmarkzellen, nach Zugabe von 20% Ueberstand aus während 24 Stunden mit Substanz inkubierten Makrophagenkulturen, im Vergleich zu 0—5 Kolonien bei Zugabe von Ueberständen unbehandelter Makrophagenkulturen). CSA ist ein biologischer Mediator, der für die Differenzierung von Knochenmark-Stammzellen zu Makrophagen und polymorphkernigen Leucocyten notwendig ist. Damit bewirken die genannten Verbindungen einen erhöhten Nachschub von Zellen, die für die unspezifische Resistenz und für die Induktion, Amplifikation und Expression spezifischer (lymphocytenvermittelter) Immunreaktionen von zentraler Bedeutung sind.

Die immunpotenzierende Wirking der neuen Verbindungen der Formel (I), ihrer Salze oder Komplexe oder Gemische, kann auch in vivo nachgewiesen werden: So führt die Injektion eines Lipopeptids gemäss der Erfindung innert 3—9 Stunden zu einem hohen Anstieg der CSA-Konzentration im Serum (bis zu 120 Kolonien pro $10^5$ Mäuseknochenmarkzellen nach Zugabe von Chloroform extrahiertem Serum [5% Endkonzentration] im Vergleich zu 0—5 Kolonien bei unbehandelten Tieren). Dementsprechend wird durch Verabreichung derselben Verbindungen in vivo die Antikörperbildungsfähigkeit von Mäusen erheblich potenziert:

NMRI Mäuse werden dirch intraperitoneale Injektion von 10 μg präzipitatfreiem BSA am Tag 0

immunisiert. 9,15 und 29 Tage später werden Serumproben entnommen und auf ihren Gehalt an anti-BSA-Antikörpern mit einer passiven Haemagglutinationstechnik untersucht. In der verwendeten Dosis ist lösliches BSA für die Empfängertiere subimmunogen, d.h. es vermag keine oder nur eine ganz geringfügige Produktion von Antikörpern auszulösen. Zusätzliche Behandlung der Mäuse mit gewissen immunpotenzierenden Stoffen vor oder nach der Antigengabe führt zu einem Anstieg der Antikörpertiter im Serum. Der Effekt der Behandlung wird durch den erreichten Scorewert, d.h. durch die Summe der $log_2$ Titerdifferenzen an den drei Blutungstagen ausgedrückt.

In diesem Test sind die Verbindungen der Formel (I), ihre Salze oder Komplexe oder Gemische in der Lage bei intraperitonealer oder subkutaner Applikation von 0,03—3 mg/kg Tier an fünf aufeinander folgenden Tagen vor oder nach Immunisierung mit BSA die Antikörperproduktion gegen BSA signifikant zu steigern.

Auch Manifestationen der zellvermittelten Immunität können durch die genannten Verbindungen in vivo potenziert werden:

Während Sensibilisierung von Meerschweinchen mit BSA in inkomplettem Freund'schen Adjuvans nur zu humoraler Antikörperbildung führt, induziert die Beimischung der Lipopeptide gemäss der vorliegenden Erfindung in einem Dosisbereich von 1—15 $\mu$g zur wässerigen Phase der Antigen-Oelemulsion Spättyp-Ueberempfindlichkeit gegenüber BSA: 3 Wochen nach Immunisierung führt die intrakutane Injektion von BSA bei diesen Tieren zu einer lokalen Entzündungsreaktion mit Erythem und Verdickung der Haut, die innert 24 bis 48 Stunden ihr Maximum erreicht. Diese Spättyp-Reaktionen entsprechen quantitativ und qualitativ denjenigen, die üblicherweise durch Immunisierung mit BSA in komplettem Freund' schem Adjuvans (d.h. mit Zusatz von Mykobakterien) erhalten werden. Die $ED_{50}$-Werte (benötigte $\mu$g/Tier zur Induktion einer Differenz des Reaktionsvolumens (Erythemfläche x Hautdickenzunahme) bei behandelten und unbehandelten Tieren von 200 $\mu$l, 24 Stunden nach Auslösung) betragen 2—3 $\mu$g.

Die Lipopeptide gemäss der vorliegenden Erfindung sind zudem wenig toxisch: Auch 5-Malige intraperitoneale Applikation in einer Dosis von 10 mg/kg/Tag an fünf aufeinanderfolgenden Tagen wurde von Mäuse anscheinend symptomlos vertragen. Da die für die Immunstimulation benötigten Dosen sehr gering sind, ist die therapeutische Breite der neuen Verbindung sehr gross.

Die neuen Lipopeptide gemäss der vorliegenden Erfindung können somit die zelluläre und besonders die humorale Immunität erheblich steigern, und zwar sowohl in Mischung mit dem Antigen selber (Adjuvanseffekt im engeren Sinne) als auch bei zeitlich und örtlich von der Antigeninjektion getrennter Zufuhr (systemische Immunpotenzierung).

Die neuen Lipopeptide gemäss der vorliegenden Erfindung können somit als Adjuvantien in Mischung mit Impfstoffen dazu benützt werden, den Impferfolg zu verbessern und den durch humorale Antikörper und/oder zelluläre Immunität vermittelten Infektionsschutz gegenüber bakteriellen, viralen oder parasitären Erregern zu verbessern.

Schliesslich eignen sich die beschriebenen Verbindungen in Mischung mit verschiedenen Antigenen als Adjuvantien bei der experimentellen und industriellen Herstellung von Antiseren für Therapie und Diagnostik und bei der Induktion von immunologisch aktivierten Lymphocytenpopulationen für Zelltransferverfahren.

Darüberhinaus können die neuen Lipopeptide auch ohne gleichzeitige Antigenzufuhr dazu benützt werden, bereits unterschwellig ablaufende Immunreaktionen bei Mensch und Tier zu fördern. Die Verbindungen eignen sich demnach besonders für die Stimulation der körperlichen Abwehr, z.B. bei chronischen und akuten Infektionen oder bei selektiven (antigenspezifischen) immunologischen Defekten, sowie bei angeborenen, aber auch bei erworbenen allgemeinen (d.h. nicht antigenspezifischen) immunoligischen Defektzuständen, wie sie im Alter, im Verlauf schwerer Primärerkrankungen und vor allem nach Therapie mit ionisierenden Strahlen oder mit immunosuppressiv wirkenden Hormonen auftreten. Die genannten Stoffe können somit vorzugsweise auch in Kombination mit Antibiotika, Chemotherapeutika oder anderen Heilverfahren verabreicht werden, um immunologischen Schädigungen entgegenzuwirken. Schliesslich sind die beschriebenen Stoffe auch zur allgemeinen Prophylaxe von Infektionskrankheiten bei Mensch und tier geeignet.

Die neuen Lipopeptide können nach an sich bekannten Methoden hergestellt werden.

Nach einem bevorzugten Verfahren werden die Verbindungen der Formel (I), ihre diastereomeren Gemische und ihre Salze und Komplexe dadurch hergestellt,

a) dass man in einer Verbindung der Formel

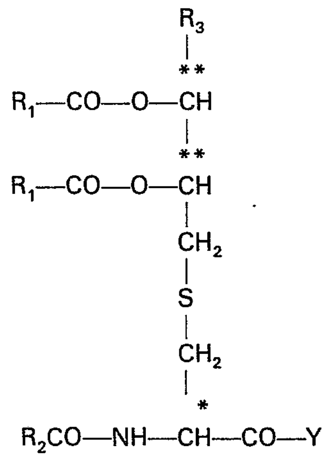

(VI)

* = R

** = R, S oder Gemisch (R,S)

worin $R_1$, $R_2$ und $R_3$ die für Formel (I) angegebene Bedeutung haben und Y eine X in der Formel (I) entsprechende Aminosäure oder Aminosäurensequenz darstellt, worin jedoch mindestens eine der die Aminosäuren substituierenden hydrophilen Gruppen und/oder die terminale Carboxylgruppe durch eine unter neutralen oder milden sauren Bedingungen abspaltbare Schutzgruppe geschützt ist, oder in einem Salz einer solchen Verbindung, die Schutzgruppe(n) abspaltet, oder

b) dass man eine Verbindung der Formel

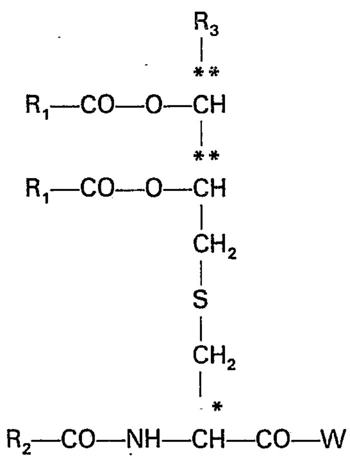

(VII)

* = R

** = R, S oder Gemisch (R,S)

worin $R_1$, $R_2$ und $R_3$ die für Formel (I) angegebene Bedeutung haben und W = OH oder eine Aminosäure oder eine unvollständige Sequenz von Aminosäuren gemäss X in Formel (I) darstellt, mit einer Verbindung der Formel

$$NH_2—Z_1 \qquad (VIII)$$

wo $Z_1$ eine der Gruppe X in Formel (I) entsprechende Gruppe bzw. eine zur genannten Aminosäure oder unvollständigen Sequenz von Aminosäuren gemäss X komplementären Aminosäuresequenz bzw. Aminosäure bedeutet, in welchen Sequenzen jedoch keine freien Aminogruppen vorhanden sind, oder einem Salz derselben, umsetzt, oder

c) eine Verbindung der Formel

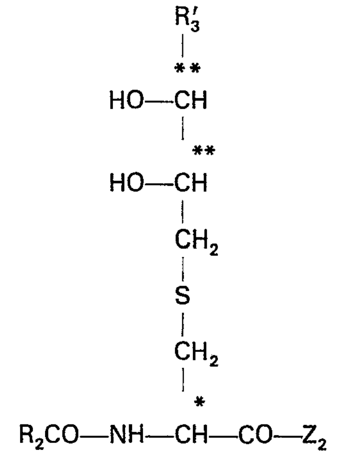

(IX)

* = R

** = R, S oder Gemisch (R,S)

worin $R_2$ die für Formel (I) Angegebene Bedeutung besitzt und $R_3$ Wasserstoff oder die Gruppe HO—$CH_2$ darstellt, und $Z_2$ eine X in Formel (I) entsprechende Gruppe darstellt, worin jedoch keine freien Hydroxygruppen vorhanden sing, oder ein Salz dieser Verbindung, in an sich bekannter Weise acyliert, und, wenn erwünscht, in erhaltenen Verbindungen mit freier terminaler Carboxylgruppe dieselbe in eine Amidgruppe oder Estergruppe überführt, und/oder die Verbindungen in ihre Salze oder Komplexe überführt.

Die Schutzgruppen im Verfahren gemäss Variante a) sind besonders die von der Synthese von Peptiden her bekannten.

So sind z.B. Schutzgruppen für Aminogruppen Acyl- oder Aralkylgruppen wie Formyl-, Trifluoracetyl-, Phthaloyl-, Benzolsulfonyl-, p-Toluolsulfonyl-, o-Nitrophenylsulfenyl-, 2,4-Dinitrophenylsulfenylgruppen (diese Sulfenylgruppen können auch durch Einwirking nucleophiler Reagenzien, z.B. Sulfite, Thiosulfate, abgespalten werden), gegebenenfalls substituierte, wie z.B. durch Niederalkoxygruppen, besonders o- oder p-Methoxygruppen substituierte Benzyl-, oder Diphenyl- oder Triphenylmethylgruppen oder von der Kohlensäure sich ableitende Gruppen, wie gegebenenfalls in den aromatischen Ringen, z.B. durch Halogenatome wie Chlor oder Brom, Nitrogruppen, Niederalkyl- oder Niederalkoxygruppen oder farbgebende Gruppen, z.B. Azogruppen substituierte Arylmethyloxycarbonylgruppen, in denen die Methylengruppe durch einen weiteren Arylrest und/oder einen oder gegebenenfalls zwei niedere Alkylreste substituiert sein kann, wie Benzyl-, Benzhydryl- oder 2-Phenyl-isopropyloxycarbonylgruppen, z.B. Carbobenzoxy, p-Brom- oder p-Chlorcarbobenzoxy, p-Nitrocarbobenzoxy oder p-Methoxycarbobenzoxy, p-Phenylazo-benzyloxycarbonyl und p-(p'-Methoxy-phenylazo)-benzyloxycarbonyl, 2-Tolyl-isopropyloxycarbonyl und insbesondere 2-(p-Biphenylyl)-isopropyloxycarbonyl, sowie aliphatische Oxycarbonylgruppen wie Adamantyloxycarbonyl, Cyclopentyloxycarbonyl, Trichloräthyloxycarbonyl, tert. Amyloxycarbonyl oder vor allem tert.-Butyloxycarbonyl.

Die Aminogruppen können auch durch Bildung von Enaminen, erhalten durch Reaktion der Aminogruppe mit 1,3-Diketonen, z.B. Benzoylaceton, Acetylaceton oder Dimedon, geschützt werden.

Carboxylgruppen sind beispielsweise durch Amid- oder Hydrazidbildung oder durch Veresterung geschützt. Die Amid- und Hydrazidgruppen können gegebenenfalls substituiert sein, die Amidgruppe z.B. durch die 3,4-Dimethoxybenzyl- oder bis-(p-Methoxyphenyl)-methylgruppe, die Hydrazidgruppe z.B. durch die Carbobenzoxygruppe, die Trichloräthyloxycarbonylgruppe, die Trifluoracetylgruppe, die Tritylgruppe, die tert.-Butyloxycarbonylgruppe oder die 2-(p-Biphenylyl)-isopropyloxycarbonylgruppe. Zur Veresterung geeignet sind z.B. niedere gegebenenfalls substituierte Alkanole wie Methanol, Aethanol, Cyanmethylalkohol, Benzoylmethylalkohol oder insbesondere tert.-Butanol, ferner Aralkanole wie Arylniederalkanole, z.B. gegebenenfalls durch Niederalkyl- oder Niederalkoxygruppen oder Halogenatome substituierte Benzylalkohole oder Benzhydrole wie Benzhydrol, p-Nitrobenzylalkohol, p-Methoxybenzylalkohol, 2,4,6-Trimethylbenzylalkohol, gegebenenfalls durch elektronenanziehende Substituenten substituierte Phenole und Thiophenole wie Thiophenol, Thiokresol, p-Nitrothiophenol, 2,4,5- und 2,4,6-Trichlorphenol, Pentachlorphenol, p-Nitrophenol, 2,4-Dinitrophenol, p-Cyanophenol oder p-Methansulfonylphenol, weiter z.B. N-Hydroxysuccinimid, N-Hydroxyphthalimid, N-Hydroxypiperidin, 8-Hydroxychinolin.

Die Hydroxygruppen der Serin- und Threoninreste können z.B. durch Veresterung oder Verätherung geschützt sein.

Als Acylreste bei der Veresterung sind vor allem von der Kohlensäure sich ableitende Reste wie Benzoyloxycarbonyl oder Aethyloxycarbonyl geeignet. Zur Verätherung geeignete Gruppen sind z.B. Benzyl-, Tetrahydropyranyl- oder tert.-Butylreste, Ferner eignen sich zum Schutz der Hydroxylgruppen

die in Ber. *100* (1967), 3838—3849 beschriebenen 2,2,2-Trifluor-1-tert.-butyloxycarbonylamino- oder -1-benzyloxycarbonylaminoäthylgruppen (Weygand).

Vorzugsweise verwendet man zum Schutz der Carboxylgruppe der Seitenketten und gegebenenfalls der terminalen Carboxylgruppe die tert.-Butylestergruppe oder die Benzhydrolgruppe, zum Schutz der Aminogruppen der Seitenketten die tert.-Butyloxycarbonylgruppe, für die Hydroxylgruppen von Serin oder Threonin, die tert.-Butyläthergruppe, und, wenn erwünscht, zum Schutz der Iminogruppe des Histidins die 2,2,2-Trifluor-1-tert.-butyloxycarbonylaminoäthylgruppe.

Die verfahrensgemässe Abspaltung der Schutzgruppen mit sauren Mitteln unter milden Bedingungen erfolgt z.B. in einer von der Peptidchemie her bekannten Weise, z.B. durch Behandlung mit Trifluoressigsäure.

Eine besondere Schutzgruppe für Carboxylgruppen, welche unter neutralen Bedingungen abspaltbar ist, ist die z.B. in der Deutschen Offenlegungsschrift 27 06 490 beschriebene Gruppe der allgemeinen Formel

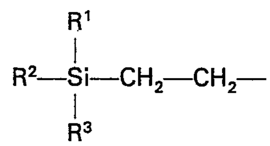

worin $R^1$, $R^2$ und $R^3$ je einen Kohlenwasserstoffrest bedeutet, wobei die Reste untereinander auch durch eine einfache C—C Bindung verknüpft sein können, insbesondere Alkylreste mit 1—5 C Atomen. Schutzgruppen dieses Typs sind z.B. die 2-(Dimethyl-tert.-butylsilyl)-äthyl-, die 2-(Dibutyl-methyl-silyl)-äthyl- und vor allem die 2-(Trimethylsilyl)-äthylgruppe. Obwohl diese Schutzgruppen auch basisch abgespalten werden können, ist besonders ihre Abspaltbarkeit unter neutralen Bedingungen, und zwar durch Einwirking eines Salzes der Fluorwasserstoffsäure, von Interesse. Die Abspaltung der Schutzgruppe wird vorteilhaft in einem aprotischen organischen Lösungsmittel vorgenommen; vorzugsweise wird dabei die Anwesenheit von solchen Lösungsmitteln vermieden, welche das Fluorid-Anion zu solvatisieren vermögen, wie Wasser oder niederaliphatische Alkohole.

Die Kondensation gemäss der Variante b) der Verbindung der Formel (VII) mit der Verbindung der Formel (VIII) erfolgt z.B. in der Weise, dass man die Verbindung (VII) in Form der aktivierten Carbonsäure mit (VIII) umsetzt, oder dass man die Säure (VII) mit der Verbindung (VIII), deren Aminogruppe in aktivierter Form vorliegt, umsetzt. Gegebenenfalls werden die Verbindungen (VII) bzw. (VIII) für die Kondensation in Form ihrer Salze eingesetzt.

Die Carboxylgruppe der Verbindung (VII) kann beispielsweise durch Ueberführung in ein Säureazid, -anhydrid, -imidazolid, -isoxazolid oder einen aktivierten Ester, wie Cyanmethylester, Carboxymethylester, Thiophenylester, p-Nitrothiophenylester, Thiokresylester, p-Methansulfonylphenylester, p-Nitrophenylester, 2,4-Dinitrophenylester, 2,4,5- oder 2,4,6-Trichlorphenylester, Pentachlorphenylester, N-Hydroxysuccinimidester, N-Hydroxyphthalimidester, 8-Hydroxychinolinester, 2-Hydroxy-1,2-dihydro-1-carbäthoxy-chinolin-ester, N-Hydroxypiperidinester oder einen Enolester, der mit N-Aethyl-5-phenyl-isoxazolium-3-sulfonat gewonnen wird [Woodward Reagens], oder durch Reaktion mittels eines Carbodiimids (gegebenenfalls unter Zusatz von N-Hydroxysuccinimid) oder eines unsubstituierten oder z.B. durch Halogen, Methyl oder Methoxy substituierten 1-Hydroxybenzotriazols, 3-Hydroxy-4-oxo-3,4-dihydrobenzo[d]-1,2,3-triazins oder N,N'-Carbonyldiimidazols, aktiviert werden.

Die Aminogruppe der Verbindung (VIII) kann beispielsweise durch Reaktion mit einem Phosphit aktiviert werden.

Als gebräuchlichste Methoden der Kondensation sind zu nennen: die Methode nach Weygand-Wünsch (Carbodiimid in Gegenwart von N-Hydroxysuccinimid), die Azidmethode, die Methode der N-Carboxyanhydride oder N-Thiocarboxyanhydride, die Methode der aktivierten Ester und die Anhydridmethode. Insbesondere können diese Kondensationen nach der Merrifield-Methode vollzogen werden.

Es ist auch möglich, die Kondensation der Verbindung (VII) nach den eben erwähnten Methoden mit einer Verbindung entsprechend der Formel (VIII) zu vollziehen, in welcher die terminale Carboxylgruppe durch eine von einer, wie für X definiert, Amid- oder Estergruppe verschiedene Schutzgruppe, wie eine der oben angegebenen, blockiert is, und/oder eine oder mehrere der vorhandenen hydrophilen Gruppen in geschützter Form, wie oben beschrieben, vorliegen. Man gelangt so zu Kondensationsprodukten, welche zur Gruppe der für die oben beschriebene Variante a) benützten Ausgangsstoffe der Formel (VI) gehören, die ebenfalls Gegenstand der vorliegenden Erfindung sind.

Gemäss der obigen Variante c) werden in Verbindungen der Formel (IX), die im Teil $Z_2$ keine freien Hydroxygruppen enthalten dürfen, die freien Hydroxylgruppen im Glyzeryl- bzw. Erythrityl-Rest acyliert. Diese acylierung kann in an sich bekannter Weise, z.B. durch Reaktion mit einem reaktiven funktionellen Derivat der dem einzuführenden Rest entsprechenden Säure, wie dem Anhydrid oder einem Säurehalogenid, vorzugsweise in Gegenwart einer tertiären Base, wie Pyridin oder Kollidin, ausgeführt werden.

Dieses Verfahren ist besonders zur Herstellung von Verfahrensprodukten gemäss Formel I, in welchen die Acylreste im Glyzeryl-bzw. Erythrityl-Rest und am Cystein-rest verschieden sind, geeignet.

Wendet man diese Variante bei Verbindungen gemäss Formel (IX) an, bei denen jedoch im Teil $Z_2$ die terminale Carboxylgruppe, ausser als durch Amid- oder Esterrest, wie für X vorgesehen, auch durch andere Schutzgruppen geschützt ist, und/oder eine oder mehrere der vorhandenen hydrophilen Gruppen in geschützter Form, wie oben beschrieben, vorliegen, so kommt man ebenfalls zu Ausgangsverbindungen für die obige Variante a).

In den nach irgend einer der beschriebenen Verfahrensvarianten erhaltenen Lipopeptiden gemäss Formel I, in welchen eine freie terminale Carboxylgruppe im Teil X vorhanden ist, kann dieselbe in an sich bekannter Weise, z.B. nach einer der in der Peptidchemie gebräuchlichen Methoden, in die Amid- oder Estergruppe übergeführt werden.

Ein als Ausgangsstoff zu verwendendes N-Acyl- oder N,O,O-Triacyl-S-(2,3-dihydroxypropyl)-cystein gemäss Formel (VII), sofern W=OH und $R_3$=H, ist, kann in folgender Weise erhalten werden: ausgehend von 1,2,5,6-D-Mannitdiacetonid gewinnt man durch Perjodatoxydation 2-O,3-O-Isopropyliden-D-glyzerinaldehyd, reduziert die Aldehyd- zur Carbinolgruppe und verestert diese mit p-Toluolsulfonsäure. Das so erhaltene 1-O-Tosyl-2(R)-O,3-O-isopropylidenglyzerin kondensiert man mit N-Acyl-(R)-cystein in Gegenwart eines basischen Mittels, z.B. Kaliumcarbonat, und erhält so das N-Acyl-S-[2(R),3-isopropylendioxy-propyl]-(R)-cystein, das sauer, z.B. mit Essigsäure, verseift wird. Das so erhaltene N-Acyl-S-[2(R),3-dihydroxypropyl]-(R)-cystein kann, wenn erwünscht, in der 2- und 3-Stellung des Glyzerinanteils, vorzugsweise unter vorübergehendem Schutz der Carboxylgruppe, z.B. mittels der oben beschriebenen Trimethylsilyläthylgruppe oder vorzugsweise der Benzhydrylestergruppe, weiter acyliert werden.

Zur Herstellung eines als Ausgangsstoff gemäss Formel (VII) zu verwendendes N-Acyl- oder N,O,O,O-Tetraacyl-S-(2,3,4-trihydroxybutyl)-cysteins kann man analog der Methode von Beispiel 7 vorgehen.

Die zur verfahrensgemässen Herstellung der neuen Lipopeptide zu verwendenden Aminosäuren bzw. Peptide sind bekannt oder können nach an sich bekannten Methoden hergestellt werden.

Die obigen Ausgangsverbindungen (VII) für die Variante b) für den Fall, dass W = OH ist, sowie ihre diastereomeren Gemische, Salze und Komplexe besitzen selber auch eine immunpotenzierende Wirkung, wobei diese in vitro im gleichen Dosisbereich wie für die Verbindungen der Formel I und ihre Derivate angegeben, auftritt. In vivo zeigen die genannten Verbindungen im oben beschriebenen Test zur Potenzierung der humoralen Immunität bei der intraperitonealen Verabreichung 5 Tage vor der Antigengabe einen Anstieg der Antikörpertiter im Serum im Dosenbereich von 1.0 bis 3 mg/kg Tier.

Diese Verbindungen können durch Acylierung einer Verbindung gemäss Formel (IX) in der jedoch anstelle von $Z_2$ eine Hydroxygruppe vorhanden ist, wie oben für Variante c) beschrieben, erhalten werden. Die Herstellung der dazu benötigten Ausgangsstoffe, d.h. die Glyzeryl- bzw. Erithrityl-N-Acylcysteine ist oben beschrieben. Verbindungen der Formel (IX), in denen $Z_2$ eine der Gruppe X in Formel (I) entsprechende Bedeutung hat, und in der aber keine freien Hydroxylgruppen vorhanden sind, können durch Umsetzung der soeben genannten N-Acyl-cystein-Derivate mit einer Verbindung der Formel $NH_2$—$Z_2$ nach den gleichen Prinzipien wie für die oben beschriebene Variante b) des allgemeinen Verfahrens zur Darstellung der erfindungsgemässen Lipopeptide angegeben, erhalten werden.

Die erhaltenen Lipopeptide können in an sich bekannter Weise in ihre Salze übergeführt werden, z.B. durch Umsetzen erhaltener saurer Verbindungen mit Alkali- oder Erdalkalihydroxyden oder erhaltener basischer Verbindungen mit Säuren.

Infolge der engen Beziehung zwischen den neuen Verbindungen in freier Form und in Form ihrer Salze und Komplexe sind im vorausgegangenen und nachfolgend unter den freien Verbindungen sinn- und zweckgemäss gegebenenfalls auch die entsprechenden Salze zu verstehen.

Erhaltene Isomerengemische können auf Grund der physikalisch-chemischen Unterschiede der Bestandteile in bekannter Weise aufgetrennt werden, beispielsweise durch Chromatographie und/oder fraktionierte Kristallisation. Vorteilhafterweise isoliert man das wirksamere der Isomeren.

Die oben beschriebenen Verfahren werden z.B. nach an sich bekannten Methoden durchgeführt, in Abwesenheit oder vorzugsweise in Anwesenheit von Verdünnungs- oder Lösungsmitteln, wenn notwendig, unter Kühlen oder Erwärmen, unter erhöhtem Druck und/oder in einer Inertgas, wie Stickstoffatmosphäre. Dabei sind unter Berücksichtigung aller im Molekül befindlichen Substituenten, wenn erforderlich, insbesondere bei Anwesenheit leicht hydrolysierbarer O-Acylreste, besonders schonende Reaktionsbedingungen, wie kurze Reaktionszeiten, Verwendung von milden sauren Mitteln in niedriger Konzentration stöchiometrische Mengenverhältnisse, Wahl geeigneter Katalysatoren, Lösungsmittel, Temperatur und/oder Druckbedingungen, anzuwenden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder einen Aufgangsstoff unter den Reaktionsbedingungen bildet oder in Form eines reaktions fähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Die vorliegende Erfindung betrifft ebenfalls pharmzeutische Präparate, welche die beschriebenen neuen Lipopeptide gemäss der Erfindung, sowohl solche entsprechend der Formel (I) wie solche der Formel (VII), ihre Gemische, Salze oder Komplexe enthalten. Bei den erfindungsgemässen pharmzeutischen Präparaten handelt es sich um solche zur enteralen, wie oralen oder rektalen, sowie parenteralen Verabreichung an Warmblüter, welche den pharmkologischen Wirkstoff allein oder zusammen mit einem pharmazeutisch anwendbaren Trägermaterial enthalten. Die Dosierung des Wirkstoffes hängt von der Warmblüter-Spezies, dem Alter und dem individuellen Zustand, sowie von der Applikationsweise ab.

Die neuen pharmazeutischen Präparate enthalten von etwa 10% bis etwa 95%, vorzugsweise von etwa 20% bis etwa 90% des Wirkstoffs. Erfindungemässe pharmazeutische Präparate können z.B. in Dosiseinheitsform, wie Dragées, Tabletten, Kapseln, Suppositorien oder Ampullen vorliegen.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropyl-methylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls magensaftresistenten Ueberzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Die nachfolgenden Beispiele illustrieren die oben beschrieben Erfindung; sie sollen jedoch diese in ihrem Umfang in keiner Weise einschränken. Temperaturen werden in Celsiusgraden angegeben. Die benützten Abkürzungen bedeuten:

| | |
|---|---|
| Z | = Carbobenzoxy |
| $Bu^t$ | = tert Butyläther |
| O $Bu^t$ | = tert. Butylester |
| BOC | = tert. Butoxycarbonyl |
| DMF | = Dimethylformamid |
| ONp | = p-Nitrophenylester |
| HOBt | = N-Hydroxybenzotriazol |
| DC | = Dünnschichtchromatographie |

In DC verwendet man Kieselgel als Adsorbens und folgende Systeme als Laufmittel:

System 3: Essigester-Pyridin-Wasser (65:20:15)
System 157: Chloroform-Methanol-Wasser-Eisessig (70:42:10:0.5)
System 157c: Chloroform-Methanol-Wasser-Eisessig (75:25:5:0.5)

## Beispiel 1

1,13 g [N-Palmitoyl-S-2(R),3-dipalmitoyloxypropyl]-Cys-Ser($Bu^t$)-Ser($Bu^t$)-Asn-OBut werden in 5 ml 90 proz. Trifluoressigsäure aufgenommen und nach 45 Minuten bei 20° die Lösung auf ca. 2 ml eingeengt. Das Produkt wird durch Zugabe von 75 ml peroxidfreiem Aether ausgefällt, abfiltriert und über Kaliumhydroxid getrocknet. Das so erhaltene Lipopeptid[N-Palmitoyl-S-2(R),3-dipalmitoyloxypropyl]-Cys-Ser-Ser-Asn-OH zeigt im Dünnschichtchromatogramm auf Silicagel folgende RF-Werte: Rf (Chloroform-Methanol-Eisessig-Wasser 79:25:0,5:4,5) = 0,25.

0.89 λ [N-Palmitoyl-S-2(R),3-dihydroxypropyl]-Cys-Ser($Bu^t$)-Ser($Bu^t$)-Asn-O$Bu^t$ werden in 10 ml Pyridin aufgenommen, 0,725 ml Palmitinsäurechlorid zugegeben und die Lösung 24 Stunden bei 45° gehalten. Man gibt dann 200 ml Chloroform zu und extrahiert die Lösung mit IN Zitronensäure, IN Natriumbicarbonat und Wasser, trocknet die organische Phase über Natriumsulfat und dampft das Lösungsmittel ab. Der Rückstand wird durch Chromatographie an Kieselgel im System Chloroform, bzw. Chloroform-Methanol (98:2) gereinigt. Das dünnschichtchromatographisch einheitliche Lipopeptid [N-Palmitoyl-S-2(R),3-dipalmitoyloxypropyl)-Cys-Ser($Bu^t$)-Ser($Bu^t$)-Asn-O$Bu^t$ zeigt im System Chloroform-Methanol (9:1) auf Silikagel einen Rf-Wert von 0,75.

Zu 0,90 g [N-Palmitoyl-S-2(R),3-dihydroxypropyl]-Cystein und 0,99 g H-Ser($Bu^t$)-Ser($Bu^t$)-Asn-O$Bu^t$ in 20 ml Dimethylformamid werden bei 0° 0,472 g Dicyclohexylcarbodiimid und 0,309 λ N-Hydroxybenzotriazol gegeben. Nach 2 Stunden bei 0° und 15 Stunden bei 20° wird abfiltriert und das Filtrat eingedampft. Den Rückstand reinigt man durch Chromatographie an einer Säule von Silikagel im

System Chloroform-Methanol (98—2). Im Dünnschichtchromatogramm auf Silikagel zeigt das so erhaltene Lipopeptid-derivat [N-Palmitoyl-S-2(R),3-dihydroxypropyl]-Cys-Ser($Bu^t$)-Ser($Bu^t$)-Asn-O$Bu^t$ einen Rf-Wert von 0,60 (Chloroform-Methanol 8:2).

6,09 g Z-Ser($Bu^t$)-Ser($Bu^t$)-Asn-O$Bu^t$ werden in 60 ml Methanol gelöst und nach Zugabe von 0,5 g Pd-Kohle (10%ig) bei Raumtemperatur während 3 Stunden hydriert. Der Katalysator wird abfiltriert und das Filtrat eingedampft. Das Produkt fällt als weisser Schaum an. Im Dünnschichtchromatogramm auf Silikagel zeigt das Peptid H-Ser($Bu^t$)-Ser($Bu^t$)-Asn-O$Bu^t$ einen Rf-Wert von 0,41 im System Chloroform-Methanol (8:2).

4,134 g Z-Ser($Bu^t$)-OH und 4,64 g H-Ser($Bu^t$)-Asn-O$Bu^t$ werden in 50 ml Dimethylformamid gelöst und bei 0° 3,172 g Dicyclohexylcarbodiimid und 1,89 g N-Hydroxybenzotriazol zugegeben. Nach 2 Stunden bei 0° und 15 Stunden bei 20° wird der ausgefallene Dicyclohexylharnstoff abgesaugt, das Filtrat eingedampft, der Rückstand in Essigester aufgenommen und die Lösung mit IN Zitronensäure, IN Natriumbicarbonat und Wasser extrahiert, über Natriumsulfat getrocknet und eingedampft. Der Rückstand wird aus Essigester-Hexan umkristallisiert. Das erhaltene Peptid Z-Ser($Bu^t$)-Ser($Bu^t$)Asn-O$Bu^t$ hat einen Schmelzpunkt von 96—98°. Auf Silikagel: Rf (Chloroform-Methanol 95:5) = 0,25.

4,65 g Z-Ser($Bu^t$)-Asn-O$Bu^t$ werden in 50 ml Methanol in Gegenwart von 0,4 g Pd-Kohle (10%ig) hydriert. Nach Abfiltrieren des Katalysators und Eindampfen des Filtrates wird das Peptid H-Ser($Bu^t$)Asn-O$Bu^t$ als weisser Schaum erhalten. Auf Silikagel: Rf (Chloroform-Methanol 7: 3) = 0,48.

5,9 g Z-Ser($Bu^t$)-OH und 3,76 g H-Asn-O$Bu^t$ werden in 60 ml Dimethylformamid gelöst und bei 0° mit 3,06 g N-Hydroxybenzotriazol und 4,53 g Dicyclohexylcarbodiimid versetzt. Nach 2 Stunden bei 0° und 15 Stunden bei 20° filtriert man, dampft das Filtrat ein und kristallisiert den Rückstand aus Essigester-Petroläther. Das erhaltene Peptid Z-Ser($Bu^t$)-Asn-O$Bu^t$ schmilzt bei 114—115°. $[\alpha]_D^{20} = +4°$ (C = 1,5 in Methanol). Auf Silikagel: Rf (Chloroform-Methanol 9:1) = 0,48.

Beispiel 2

1,6 g [N-Palmitoyl-S-2(R),3-dipalmitoyloxypropyl]-Cys-Ser($Bu^t$)-Ser($Bu^t$)-Asn-Ala-O$Bu^t$ werden in 7,5 ml 90%iger-Trifluoroessigsäure gelöst und die Lösung nach 15 Minuten bei 20° auf ca. 2 ml eingeengt. Man fällt das Produkt mit 100 ml Aether aus, filtriert und trocknet über Natriumhydroxyd. Es resultiert ein weisses Pulver vom Schmelzpunkt 215—217° (Zersetzung), das das Lipopeptid [N-Palmitoyl-S-2(R),3-dipalmotoyloxypropyl]-Cys-Ser-Ser-Asn-Ala-OH darstellt. Auf Silikagel: Rf (157) = 0,55.

Beispiel 3

0,95 g [N-Palmitoyl-S-2(R),3-dipalmotoyloxypropyl]-Cys-Ser($Bu^t$)-Ser($Bu^t$)-O$Bu^t$ werden in 4 ml 90%iger Trifluoressigsäure gelöst. Nach 15 Minuten bei 20° gibt man 100 ml Aether zu, filtriert ab und trocknet den Rückstand über Natriumhydroxyd. Das erhaltene Lipopeptid [N-Palmitoyl-S-2(R),3-dipalmotoyloxypropyl]Cys-Ser-Ser-OH wird als farbloses, nicht hygroskopisches Pulver erhalten. Auf Silikagel: Rf (Chloroform-Methanol-Eisessig-Wasser 79:25:0,5:4,5) = 0,53.

Beispiel 4

Das in Beispiel 1 als Ausgangsstoff verwendete N-Palmitoyl-S-2(R),3-dihydroxypropyl]-(R)-cystein kann folgendermassen hergestellt werden:

13,9 g N-Palmitoyl-(R)-cystein, 5,5 g Kaliumcarbonat und 15 g 1-O-Tosyl-2(R)-O-3-O-isopropyliden-glyzerin erhitzt man 8 Stunden bei 80° in 250 ml Aethanol unter Stickstoff. Man reinigt das nach dem Eindampfen erhaltene Reaktionsgemisch durch Chromatographie an 400 g Kieselgel Merck durch Elution, zuerst mit Chloroform/Aceton (8:2), dann mit Chloroform/Methanol (8:2) Man erhält so das Kaliumsalz des N-Palmitoyl-S-[2(R),3-isopropylidendioxypropyl]-(R)-cystein, das durch 4-stündiges Erhitzen auf 80° in 80% wässriger Essigsäure verseift wird. Man dampft zur Trockne, löst den Rückstand in Chloroform und schüttelt mit Wasser aus. Nach Trocknen und Eindampfen der Chloroform-Phase erhält man einen farblosen Rückstand, der aus Cyclohexan umkristallisiert wird und das N-Palmitoyl-S-[2(R),3-dihydroxypropyl]-(R)-cystein darstellt. Fp. 110°, $[\alpha]_D^{20} = -25°$ (c = 0.9, MeOH).

Das N-Palmitoyl-(R)-cystein kann folgendermassen hergestellt werden:

45 g (R)-Cystein (0,37 mol) suspendiert man in 350 ml Pyridin und tropft unter gutem Rühren und in einer Stickstoffatomosphäre bei Raumtemperatur eine Lösung von 140 ml (1,67 Aequivalente) Palmitinsäurechlorid in 550 ml Methylenchlorid zu. Nach 20-stündigem Rühren säuert man mit 2N-Salzsäure an und verteilt zwischen Chloroform und Wasser. Nach Trocknen und Eindampfen der Chloroform-Phase erhält man ein Kristallgemisch, bestehend aus etwa einem Drittel Dipalmitoylcystein und etwa zwei Drittel Monopalmitoylcystein. Man extrahiert dreimal mit 1 Liter heissem Aceton, aus dem ein 1 : 1-Gemisch von Di- und Mono-Palmitoylcystein auskristallisiert. Aus der Acetonlösung erhält man durch Eindampfen N-Palmitoylcystein, das aus Benzin umkristallisiert wird. Fp 65—67°. $[\alpha]_D^{20} = +1°$ (c = 0,8; Methanol).

Die Mischungen aus Di- und Mono-palmitoyl-cystein werden in methanolischer Lösung unter Zugabe einer wässrigen Lösung von 12 g Natriumsulfid mit 2 ml conc. Natronlauge 15 Minuten bei Raumtemperatur behandelt. Nach Abdampfen des Methanols und Ansäueren des Rückstandes mit 2N-Salzsäure erhält man durch Chloroform-Extraktion das restliche N-Palmitoyl-cystein, das ebenfalls aus Benzin umkristallisiert wird.

Beispiel 5

0,80 g N-Palmitoyl-S-[2(R),3-dipalmitoyloxypropyl]-(R)-cystein und 0,67 g H-Ser(Bu$^t$)-Ser(Bu$^t$)-Phe-Ala-Glu(OBu$^t$)$_2$ werden in 10 ml Dimethylformamid aufgenommen und 0,22 g Dicyclohexylcarbodiimid und 0,16 g N-Hydroxybenzotriazol zugegeben. Durch kurzes Erwärmen auf 40° wird eine klare Lösung erhalten, die nach ca. einer Stunde bei 20° zu einer Gallerte erstarrt. Letztere wird nach 24 Stunden durch Erwärmen auf 40° wieder in Lösung gebracht und daraus das N-Palmitoyl-S-[2(R),3-dipalmitoyloxypropyl]-Cys-Ser(Bu$^t$)-Ser(Bu$^t$)-Phe-Ala-Glu(OBu$^t$)$_2$ mit Wasser ausgefällt. Das Produkt wird durch Chromatographie an Kieselgel im system Chloroform bzw. Chloroform-Methanol 98:2 gereinigt und besitzt dann den Rf-Wert 0,64 in Chloroform-Methanol-Wasser 95:5.

540 mg der so erhaltenen Verbindung werden in 30 ml 90%iger Trifluoressigsäure gelöst und die Lösung nach 30 Minuten bei 20° auf ungefähr die Hälfte eingeengt und mit Petroläther das N-Palmitoyl-S-[2(R),3-dipalmitoyloxypropyl]-Cys-Ser-Ser-Phe-Ala-Glu-OH ausgefällt. Die Verbindung wird abzentrifugiert, dreimal mit Petroläther gewaschen und über Kaliumhydroxid getrocknet. Sie stellt ein weisses Pulver mit dem Rf-Wert (System 157) = 0,63 dar.

In analoger Weise werden folgende Lipopeptide hergestellt:

N-Palmitoyl-S-[2(R,S),3-dipalmitoyloxypropyl]-Cys-Phe-Phe-Asn-Ala-Lys-OH, Rf (System 157 c) = 0,18;
N-Myristoyl-S-[2(R),3-dipalmitoyloxypropyl]-Cys-Glu-Gln-Asn-Ala-Lys-OH, Rf (System 157 c) = 0,12;
N-Lauroyl-S-[2(R),3-dipalmitoyloxypropyl]-Cys-Ser-Ser-Asn-Ala-Glu-OH, Rf (System 157 c) = 0,20;
N-Stearoyl-S-[2(R),3-dipalmitoyloxypropyl]-Cys-Ser-Ser-Asn-Ala-Ala-OH, Rf

Das oben beschriebene mit den genannten Schutzgruppen versehene Peptid, das als Ausgangsstoff für die Kondensation mit N-Palmitoyl-S-[2(R),3-dipalmitoyloxypropyl]-(R)-Cystein verwendet wird, kann folgendermassen hergestellt werden:

17,22 g Z-Ala-ONp, 14,8 g HCl. HGlu(OBu$^t$)$_2$ und 6,3 ml N-Ethylmorpholin werden in 40 ml Dimethylformamid über Nachtbei 20° stehen gelassen. Die Lösung wird eingedampft, der Rückstand in Essigester aufgenommen und mit 1-N-Natriumbicarbonat und Wasser extrahiert.

Die Essigesterlösung wird eingedampft und es hinterbleibt als Schaum das Z-Ala-Glu(OBu$^t$)$_2$ mit dem Rf-Wert 0,60 (in Chloroform-Methanol 95:5), das direkt weiterverarbeitet wird: 8,5 g werden in 60 ml Methanol gelöst und nach Zugabe von 0,8 mg Pd-Kohle (10%-ig) bei 20° während 2 Stunden hydriert. Der Katalysator wird abfiltriert und das Filtrat eingedampft. Man erhält das H-Ala-Glu(OBu$^t$)$_2$ als weissen Schaum, das den Rf 0.36 in Chloroform-Methanol 9:1 aufweist.

Zu 11,46 g Z-Phe-OH und 12,64 g H-Ala-Glu(OBu$^t$)$_2$ in 50 ml Dimethylformamid gibt man bei 0° 8,68 g Dicyclohexylcarbodiimid und 5,86 g N-Hydroxybenzotriazol. Nach 15 Stunden bei 4° wird abfiltriert, das Filtrat eingedampft und der Rückstand aus Essigester-Hexan umkristallisiert. Man erhält das Z-Phe-Ala-Glu(OBu$^t$)$_2$ vom Smp. 161—163°. Rf (Toluol-Aceton 1:1) = 0,63.

6,4 g dieses Produkts werden in 50 ml Methanol gelöst und nach Zugabe von 0,5 g Pd-Kohle (10%-ig) bei 20° während 20 Minuten hydriert. Der Katalysator wird abfiltriert und das Filtrat eingedampft, wobei das H-Phe-Ala-Glu(OBu$^t$)$_2$ als weisser Schaum erhalten wird. Rf (Chloroform-Methanol 7:3) = 0,75.

Zu 4,43 g Z-Ser(Bu$^t$)-OH und 3,18 g HCl.H-Ser(Bu$^t$)-OMe in 30 ml Dimethylformamid gibt man bei 0° 1,89 ml N-Ethylmorpholin, 2,29 g HOBt und 3,4 g Dicyclohexylcarbodiimid. Nach 2 Stunden bei 0° und 15 Stunden bei 20° wird abfiltriert, das Filtrat eingedampft und in Essigester aufgenommen. Nach Extraktion der Lösung mit Natriumbicarbonat, verdünnter Salzsäure und Wasser dampft man ein, wobei das Z-Ser(Bu$^t$)-Ser(Bu$^t$)-CMe als Oel erhalten wird. Rf (Toluol-Aceton 1:1) = 0,70.

Zu einer Lösung von 6,8 g dieses Produkts in 40 ml Methanol werden 7,5 ml Hydrazinhydrat gegeben und nach 24 Stunden bei 20° auf etwa die Hälfte des Volumens eingedampft. Man nimmt in 250 ml Essigester auf und extrahiert mit Wasser. Die Essigesterlösung wird eingeengt und das erhaltene Z-Ser(Bu$^t$)-Ser(Bu$^t$)-NH—NH$_2$ mit Petroläther ausgefällt. Rf (Toluol-Aceton 1:1) = 0,50.

2,26 g dieses Produkts werden in 20 ml Dimethylformamid gelöst und bei —10° mit 7,022 ml 1,78 N—HCl in Essigester und 0,635 ml tert. Butylnitrit versetzt. Nach 10 Minuten bei —10° wird eine Lösung von 2,38 g H-Phe-Ala-Glu(OBu$^t$)$_2$ und 2,52 ml N-Ethylmorpholin zugetropft und eine Stunde bei —10° und 15 Stunden bei 0° gehalten. Die Lösung wird dann eingedampft, der Rückstand in Essigester aufgenommen und mit Natriumbicarbonat, verdünnter Salzsäure und Wasser gewaschen. Das nach Eindampfen der Losüng erhaltene Z-Ser(Bu$^t$)-Ser(Bu$^t$)-Phe-Ala-Glu(OBu$^t$)$_2$ wird aus Essigester-Hexan umkristallisiert. Smp. 213—215°, Rf (Toluol-Aceton 1:1) = 0,70.

2,30 g dieses Produkts werden in 40 ml Methanol gelöst und nach Zugabe von 0,5 g Pd-Kohle (10%-ig) bei 20° während 2 Stunden hydriert. Der Katalysator wird abfiltriert und das Filtrat eingedampft, wobei das H-Ser(Bu$^t$)-Ser(Bu$^t$)-Phe-Ala-Glu(OBu$^t$)$_2$ als weisser Schaum anfällt. Rf (Toluol-Aceton 1:1) = 0,23.

Das als Ausgangsstoff verwendete N-Palmitoyl-S-[2(R),3-dipalmitoyloxypropyl]-(R)-cystein und das diastereomere Gemisch dieser Verbindung mit der entsprechenden 2(S)-Verbindung, die abgekürzt

hier als 2(R,S) bezeichnet wird, können folgendermassen hergestellt werden:

6,3 g (2.78 mMol) N-Palmitoyl-S-[2(R),3-dipalmitoyloxypropyl]-(R)-cystein-benzhydrylester löst man in einer Mischung aus 12 ml Trifluoressigsäure und 48 ml Methylenchlorid. Nach einer Stunde bei Raumtemperatur dampft man die Lösung ein und verreibt den öligen Rückstand mit Wasser. Das auskristallisierte N-Palmitoyl-S-[2(R),3-dipalmitoyloxypropyl]-(R)-cystein wird abgesaugt, getrocknet und zur Entfernung des Benzhydrols dreimal mit Benzin verrieben. Man kristallisiert den Rückstand aus Aceton-Wasser und erhält so farblose Kristalle dieses Produkts vom Smp. 71—75° und $[\alpha]_D^{20} = -1°$ (Dioxan).

Den obigen Ester erhält man auf folgende Art:

12 g (20 mMol) N-Palmitoyl-S-[2(R),3-dihydroxypropyl]-(R)-cystein-benzhydrylester löst man in einer Mischung aus 60 ml Pyridin und 60 ml Methylenchlorid und tropft bei 0—10° 14,5 ml (13,2 g) Palimitinsäurechlorid zu. Nach 2 Stunden bei Raumtemperatur ist die Reaktion gemäss Dünnschichtchromatogramm beendet. Man gibt zur Suspension (Pyridinhydrochlorid) 10 ml Methanol, rührt 20 Minuten bei 25° und dampft dann das Reaktionsgemisch weitgehend ein. Man nimmt den Rückstand in Chloroform auf, schüttelt je zweimal mit 0,5 N-Salsäure, 10% Natriumbicarbonat und Wasser aus, trocknet die Chloroform-Phase mit Natriumsulfat und dampft sie zum Syrup ein. Den reinen Ester erhält man durch Chromatographie dieses Syrups über Kieselgel in Chloroform. Die chromatographisch einheitlichen Fraktionen (DC-Kontrolle auf Kieselgel in Chloroform: Essigester 98:2, Rf = 0,5) werden eingedampft und aus Aceton umkristallisiert. Man erhält farblose Kristalle des N-Palmitoyl-S-[2(R),3-dipalmitoyloxypropyl]-(R)-cystein-benzhydrylesters vom Smp. 69—71°.

Der oben verwendete Benzydrylester der N-Palmitoyl-S-[2(R),3-dihydroxypropyl]-(R)-cysteins erhält man z.B. auf folgende Weise:

23,4 g (54 mMol) N-Palmitoyl-S-[2(R),3-dihydroxypropyl]-(R)-cystein löst man in 150 ml Chloroform-Aethanol-Gemisch 1:1 und tropft dazu bei Raumtemperatur eine Lösung von 13,6 g (70 mMol) Diphenyldiazomethan in 50 ml Chloroform. Nach 3 Stunden bei Raumtemperatur ist die anfänglich rote Lösung entfärbt und nach DC (Chloroform: Methanol = 9:1, Kieselgel) die Reaktion beendet.

Man dampft im Vakuum zur Trockne und extrahiert den Rückstand kalt mit Petroläther. Der Rückstand wird über Kieselgel mit Methylenchlorid als Lösungsmittel filtriert. Man erhält so farblose Kristalle des Benzhydrylesters vom Smp. 88—91°.

Zur Herstellung des N-Palmitoyl-2-[2(R,S),3-dihydroxypropyl]-(R)-cysteins verfährt man wie folgt:

50 g (139 mMol) N-Palmitoyl-(R)-cystein, 12,4 g Glycid und 45 g Kaliumcarbonat werden in 375 ml Aethanol unter Stickstoff und gutem Rühren 16 Stunden auf 80° erhitzt. Nach dem Abkühlen auf Raumtemperatur säuert man mit 2 N-Salzsäure auf pH ca. 4 an und versetzt mit Wasser; dabei fällt das Produkt aus. Man saugt ab und wäscht mit Wasser bis das Filtrat frei von Chlorionen ist. Nach dem Trocknen des Nutschgutes im Vakuum kristallisiert man aus Essigester um. Man erhält farblose Kristalle des Diastereomerengemisches des N-Palmitoyl-S-[2(R),3-dihydroxypropyl]-(R)-cysteins und N-Palmitoyl-S-[2(S),3-dihydroxypropyl]-(R)-cysteins vom Smp. 76—155°.

Durch Umsetzung des N-Palmitoyl-2-[2(R,S),3-dihydroxypropyl]-(R)-cysteins mit Diphenyldiazomethan, wie oben für R-Verbindung angegeben S, und anschliessende Palmitoylerung ebenfalls wie eben angegeben erhält man den N-Palmitoyl-S-[2(R,S),3-dipalmitoyloxypropyl]-(R)-cystein-benzhydrylester: farblose Kristalle vom Smp. 84—85°.

Wenn man dieses Diastereomeren-Gemisch (19,8 g) an einer Säule mit 250 g Kieselgel (Merck) mit Methylenchlorid als Lösungsmittel chromatographiert, so erhält man in einer ersten Fraktion (1,55 g) das R-Diastereomere (Rf = 0,61, Methylenchlorid: Essigester = 98:2, Dünnschicht, Kieselgel), in einer zweiten Fraktion 12,7 g eines R,S-Diastereomerengemisches und in einer dritten Fraktion (1,1 g) des S-Diastereomeren (Rf = 0,53 in Methylenchlorid: Essigester = 98:2).

Aus dem (S)-Diastereomeren erhält man in der oben beschriebenen Weise das N-Palmitoyl-S-[2(S),3-dipalmitoyloxypropyl](R)-cystein; farblose Kristalle, Smp. 64—65° $[\alpha]_D^{20} = +1°$ (Dioxan).

Die für die Herstellung der in diesem Beispiel erwähnten in analoger Weise wie das N-Palmitoyl-S-[2(R),3-dipalmitoyloxypropyl]-Cys-Ser-Ser-Phe-Ala-Glu-OH herzustellenden neuen Lipopeptide gemäss der Erfindung benötigten Ausgangsstoffe sind z.B. das

N-Myristoyl-S-[2(R),3-dihydroxypropyl]-(R)-cystein, farblose Kristalle vom Smp. 140—150°, und das
N-Stearoyl-S-[2(R),3-dihydroxypropyl]-(R)-cystein, farblose Kristalle vom Smp. 140—150°;
N-Lauroyl-S-[2(R),3-dihydroxypropyl]-(R)-cystein, farblose Kristalle vom Smp. 140—150°.

Sie können gemäss den Angaben im Beispiel 4 hergestellt werden.

Die gemäss den obigen Angaben herzustellenden Benzhydrylester haben folgende Daten:

N-Myristoyl-S-[2(R),3-dihydroxypropyl]-(R)-cystein-benzhydrylester, farblose Kristalle vom Smp. 90—92°;
N-Stearoyl-S-[2(R),3-dihydroxypropyl]-(R)-cystein-benzhydrylester, farblose Kristalle vom Smp. 95°—97°;
N-Lauroyl-S-[2(R),3-dihydroxypropyl]-(R)-cystein-benzhydrylester, farblose Kristalle vom Smp. 95—97°.

In analoger Weise wie beschrieben kann man folgende N-Acyl-S-[2(R),3-diacyloxypropyl]-(R)-cystein-benzhydrylester mit verschiedenen Acylgruppen am N und an den O-Atomen erhalten:

N-Lauroyl-S-[2(R),3-dipalmitoyloxypropyl]-(R)-cystein-benzhydrylester, Smp. 65—68°;
N-Myristoyl-S-[2(R),3-dipalmitoyloxypropyl]-(R)-cystein-benzhydrylester, Smp. 68—70°;
N-Stearoyl-S-[2(R),3-dipalmitoyloxypropyl]-(R)-cystein-benzhydrylester, Smp. 70—72°.

Weitere als Zwischenprodukt zur Verwendende Verbindungen, die nach den obigen Angaben hergestellt werden können, sind:

N-Lauryl-S-[2(R),3-dipalmitoyloxypropyl]-(R)-cystein, farblose Kristalle, Smp. 70—72°;
N-Myristoyl-S-[2(R),3-dipalmitoyloxypropyl]-(R)-cystein, farblose Kristalle, Smp. 71—73°;
N-Stearoyl-S-[2(R),3-dipalmitoyloxypropyl]-(R)-cystein, farblose Kristalle, Smp. 74—77°.

Beispiel 6

Mittels der im Beispiel 5 beschriebenen Reaktionen werden folgende Ausgangsstoffe zur Herstellung von Lipopeptiden gemäss der Anmeldung hergestellt:

N-Palmitoyl-S-[2(R),3-dilauroyloxypropyl]-(R)-cystein-benzhydrylester, farbloses Wachs;
N-Palmitoyl-S-[2(R),3-distearoyloxypropyl]-(R)-cystein-benzhydrylester, farblose Kristalle, Smp. 80—82°;
N-Palmitoyl-S-[2(R),3-dioleoyloxypropyl]-(R)-cystein-benzhydrylester, farbloses Oel;
N-Palmitoyl-S-[2(R),3-dibehenoyloxypropyl]-(R)-cystein-benzhydrylester, farblose Kristalle, Smp. 85—88°;
N-Palmitoyl-S-[2(R),3-di-(dihydrosterkuloyloxypropyl]-(R)-cystein-benzhydrylester, farbloses Wachs.

Die entsprechenden nicht veresterten substituierten Cysteine haben folgenden Daten:
N-Palmitoyl-S-[2(R),3-dilauroyloxypropyl]-(R)-cystein, farbloses Oel, Rf = 0,27;
N-Palmitoyl-S-[2(R),3-distearoyloxypropyl]-(R)-cystein, farblose Kristalle, Smp. 82—85°;
N-Palmitoyl-S-[2(R),3-dioleoyloxypropyl]-(R)-cystein, farbloses Oel, Rf in Chloroform: Methanol = 9:1 (Dünnschicht- Kieselgel-Merck) = 0,35;
N-Palmitoyl-S-[2(R),3-dibehenoyloxypropyl]-(R)-cystein, farblose Kristalle, Smp. 87—90°;
N-Palmitoyl-S-[2(R),3-di-(dihydrosterkuloyloxypropyl]-(R)-cystein, farbloses Wachs, Rf = 0,38 (gleiche Bedingungen wie oben für das N-Palmitoyl-2,3-dioleoyloxy-Derivat angegeben).

Diese Cystein-Derivate werden nach den Angaben im Beispiel 5 mit folgenden Peptiden kondensiert:

H-Phe-Phe-Asn-Ala-Lys-OH
H-Glu-Gln-Asn-Ala-Lys-OH
H-Ser-Ser-Asn-Ala-Glu-OH

Man erhält so die entsprechenden Lipopeptide gemäss der Erfindung.

Beispiel 7

Man lässt 0,9 g N-Palmitoyl-S-2(R),3(R),4-tripalmitoyloxy-butyl-(R)-cystein-benzhydrylester in einer Mischung aus 3 ml Trifluoressigsäure und 12 ml Methylenchlorid 2 Stunden bei Raumtemperatur stehen. Dann dampft man zur Trockne, verreibt den Rückstand mit Eiswasser, saugt ab und trocknet den Festkörper. Dann extrahiert man ihn mit Benzin und kristallisiert aus Aceton um. Man erhält farblose Kristalle des N-Palmitoyl-S-2(R),3(R),4-tripalmitoyloxy-butyl-(R)-cysteins vom Smp. 76—76,5° und der spezifischen Drehung $[\alpha]_D^{20} = -5°$ (c = 0,819, Dioxan).

Den als Ausgangsmaterial verwendeten Benzhydrylester gewinnt man auf folgende Weise:

1 g N-Palmitoyl-S-2(R),3(R),4-trihydroxybutyl-(R)-cystein-benzhydrylester werden in einer Mischung aus 6 ml Pyridin in 5 ml Methylenchlorid mit 1,83 ml Palmitinsäurechlorid acyliert. Nach 18 Stunden bei Raumtemperatur gibt man 1 ml Methanol zu und dampft zur Trockne. Den Rückstand filtriert man über 100 g Kieselgel Merck mit Chloroform als Lösungsmittel. Die nach Dünnschichtchromatographie ($CHCl_3$: Essigester = 98:2) reinen Fraktionen geben nach Eindampfen farblose Kristalle vom Smp. 60—62°.

Das Ausgangsmaterial für die obige Reaktion gewinnt man wie folgt:

2,53 g N-Palmitoyl-S-2(R),3(R),4-trihydroxybutyl-(R)-cystein lässt man in einer Mischung aus 12 ml Aethanol und 32 ml Chloroform mit 1,37 g Diphenyldiazomethan 15 Stunden bei Raumtemperatur reagieren. Man dampft zur Trockne und reinigt den entstandenen Benzhydrylester an 150 g Kieselgel in Chloroform als Lösungsmittel. Rf = 0,3 ($CHCl_3$: Aceton = 95:5) Kieselgel Merck, Dünnschicht.

N-Palmitoyl-S-2(R),3(R),4-trihydroxybutyl-(R)-cystein gewinnt man auf folgende Weise.

20 g N-Palmitoyl-cystein, 27,6 g 1-Toxyl-2,4-äthyliden-D-erythrit und 17 g Pottasche werden

unter Stickstoff in 240 ml Aethanol 15 Stunden bei 80° gerührt. Dann filtriert man die Salze ab und dampft das Filtrat zur Trockne. Man nimmt den Rückstand in 200 ml $H_2O$: Tetrahydrofuran = 1:1 auf und säuert mit 2 N-Salzsäure auf pH = 3 an. Dann schüttelt man dreimal mit Aether aus, stellt mit Pyridin den pH der Aetherphase auf 4,5 ein, schüttelt mit Wasser aus, trocknet die Aetherphase und dampft zur Trockne. Diesen Rückstand reinigt man über 200 g Kieselgel Merck mit Chloroform: Aceton = 8:2 (1 1) und dann mit $CHCl_3$:MeOH = 6:4 (1,2 1) als Elutionsmittel.

Die reinen Fraktionen werden eingedampft, mit Hexan verrieben und aus Essigester-Petroläther umkristallisiert.

Man erhält farblose Kristalle des N-Palmitoyl-S-[(2,4-äthyliden)-erythrityl]-(R)-cysteins vom Smp. 62—65°.

Die Hydrolyse dieser Verbindung wird mit 3-proz. $HBF_4$ bei 80° während 4.5 Stunden ausgeführt.

8,2 g Aethylidenderivat werden in 100 ml Dimethoxyäthan und 100 ml 3-proz. $HBF_4$ behandelt. Dann kühlt man auf 0°, wobei das Hydrolyseprodukt ausfällt. Nach Absaugen und Trocken kristallisiert man aus Essigester um. Smp. 160—165°, Sintern ab 93°. Rf = 0,285 ($CHCl_3$:MeOH = 6:4) Dünnschicht, Kieselgel, Merck.

Beispiel 8

Analog den Angaben von Beispiel 5 werden aus N-Stearoyl-S-[2(R),3-dipalmitoyloxypropyl]-(R)-cystein und aus N-Palmitoyl-S-[2(R),3(R),4-tripalmitoyloxybutyl]-(R)-cystein folgende Lipopeptide hergestellt:

N-Stearoyl-S-[2(R),3-dipalmitoyloxypropyl]-Cys-Ser-Ser-Asn-Ala-Glu-Ile-Asp-Glu-OH, Rf (157) = 0,32;
N-Palmitoyl-S-[2(R),3(R),4-tripalmitoyloxybutyl]-Cys-Ser-Ser-Asn-Ala-Lys-OH, Rf (157) = 0,55;
N-Palmitoyl-S-[2(R),3(R),4-tripalmitoyloxybutyl]-Cys-Ser-Ser-Asn-Ala-Lys-Ile-Asp-Glu-OH, Rf (157) = 0,38.

Beispiel 9

Analog den Angaben des Beispiels 5 werden aus N-Palmitoyl-2(R),3-dipalmitoyloxypropyl-(R)-cystein oder N- Palmitoyl 2(R,S), 3-dipalmitoyloxypropyl-Cystein die nachstehend aufgeführten Lipopeptide hergestellt, wobei die Reste der eben genannten Cystein-Derivate mit PC(R) bzw. PC(R,S) bezeichnet werden:

PC(R,S)-Ser-Ser-Asn-OH
PC(R)-Ser-Ser-Asn-Ala-Lys-OH
PC(R,S)-Ser-Ser-Asn-Ala-Lys-OH
PC(R)-Ser-OH
PC(R,S)-Ser-OH
PC(R,S)-Ser-Ser-Asn-Ala-OH
PC(R)-Phe-Ile-Ile-Phe-Ala-OH
PC(R,S)-Phe-Ile-Ile-Phe-Ala-OH
PC(R)-Val-Lys-Val-Tyr-Pro-OH
PC(R,S)-Val-Lys-Val-Tyr-Pro-OH
PC(R)-Ala-Ile-Gly-Val-Gly-Ala-Pro-$NH_2$
PC(R,S)-Ala-Ile-Gly-Val-Gly-Ala-Pro-$NH_2$
PC(R)-Ser-Ser-Asn-Ala-Lys-Ile-Asp-Glu-OH
PC(R,S)-Ser-Ser-Asn-Ala-Lys-Ile-Asp-Glu-OH

Ferner stellt man die R, S-Epimerengemische sämtlicher in den vorhergehenden Beispielen beschriebenen Lipopeptide her.

**Patentansprüche**

1. Verbindungen der Formel

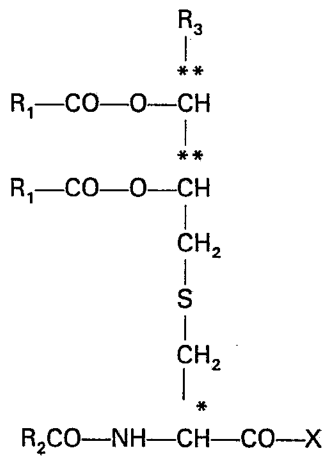

(I)

* = R

** = R oder S

worin $R_1$ und $R_2$ je einen gesättigten oder ungesättigten, aliphatischen oder gemischt aliphatischcycloaliphatischen, gegebenenfalls auch durch Sauerstoffunktionen substituierten, Kohlenwasserstoffrest mit 11—21 C-Atomen, $R_3$ Wasserstoff oder den Rest $R_1$—CO—O—$CH_2$— wo $R_1$ die gleiche Bedeutung hat, und X eine peptidisch gebundene natürliche aliphatische Aminosäure mit freier, veresterter oder amidierter Carboxylgruppe, oder eine Aminosäurensequenz von 2—10 natürlichen aliphatischen Aminosäuren, deren terminale Carboxylgruppe in freier, veresterter oder amidierter Form vorliegt, bedeuten, wobei die mit * bzw. ** bezeichneten Asymmetriezentren die absolute R- bzw. S- oder R-Konfiguration besitzen, und gegebenenfalls Gemische der an den **C-Atomen epimeren R- und S-Verbindungen, und ihre Salze und Komplexe.

2. Verbindungen gemäss Anspruch 1, worin die Acylreste $R_1CO$ und $R_2CO$ sich von gesättigten oder ungesättigten Fettsäuren mit 14—18 C-Atomen ableiten, und worin $R_1$ und $R_2$ identisch oder verschieden sein können.

3. Verbindungen gemäss Anspruch 1, worin die Acylreste sich von der Palmitin-, Stearin-, Oel-, Laurin-, Myristin-, Behen-, Dihydrosterkul-, Malval-, Hydnocarpus- oder Chaulmoograsäure ableiten.

4. Verbindungen gemäss einem der Ansprüche 1—3, worin hydrophile Gruppen tragende Aminosäuren in X die ionischen Aminosäuren Asparagin-, Glutamin- und/oder Oxyglutaminsäure, oder Lysin, Ornithin, Arginin und/oder Histidin, oder Asparagin, Glutamin, Serin und/oder Threonin sind, und keine hydrophile Gruppen tragenden Aminosäuren in X Glycin, Alanin, Valin, Norvalin, Leucin, Isoleucin und/oder Methionin sind.

5. Verbindungen gemäss einem der Ansprüche 1—4, worin in der Peptidkette X die Aminosäuren mit hydrophilem Charakter direkt miteinander verbunden sind.

6. Verbindungen gemäss Anspruch 5, worin die Sequenz der Aminosäuren mit hydrophilem Charakter direkt an die Carboxylgruppe des Cysteins im Triacyl-Glyzeryl-bzw. Tetracyl-, Erithrityl-cystein-Teil gebunden ist.

7. Verbindungen gemäss einem der Ansprüche 1—6, worin X in Formel 1 eine Aminosäure oder eine Peptidkette mit 2—5 Aminosäuren darstellt.

8. Verbindungen gemäss Anspruch 1 der Formel

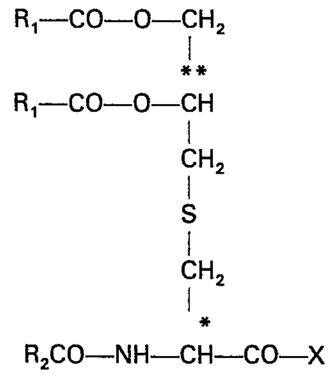

(IV)

* = R

** = R

ihre Salze und Komplexe, worin X eine peptidisch gebundene natürliche aliphatische Aminosäure mit einer hydrophilen Gruppe und freier, veresterter oder amidierter Carboxylgruppe, oder eine Aminosäuresequenz von 2—5 natürlichen aliphatischen Aminosäuren, von denen die Hälfte mindestens eine hydrophile Gruppe tragen, deren terminale Carboxylgruppe in freier, veresterter oder amidierter Form vorliegt, wobei die mit * und ** bezeichneten Asymmetrizentren die R-Konfiguration haben.

9. Verbindungen nach einem der Ansprüche 1—6, worin X in Formel I des Anspruchs 1 eine im Vergleich zu den bekannten Murein-Lipoprotein-Abbau-Lipopeptiden unnatürliche Aminosäuresequenz aufweist.

10. Verbindungen entsprechend denjenigen des Anspruchs 8, in denen jedoch die Konfiguration am C**-Atom S statt R ist, und die Gemische der R- und S-Diastereomeren.

11. Verbindungen gemäss einem der Ansprüche 1—6 der Formel

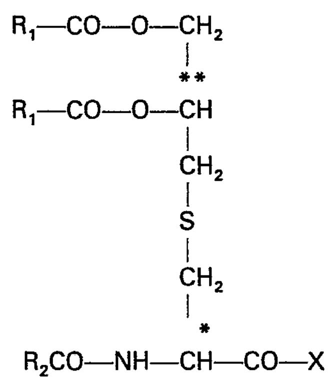

* = R

** = R

worin $R_1$ und $R_2$ die für Formel I angegebene Bedeutung haben, und X eine Peptidkette mit 6—10 Aminosäuren darstellt, wobei die mit * und ** bezeichneten Asymmetriezentren die R-Konfiguration haben, sowie die am C**-Atom die (S)-Konfiguration aufweisenden Verbindungen, sowie die R- und S-Diastereomeren-Gemische, ihre Salze und Komplexe.

12. Verbindungen gemäss einem der Ansprüche 1—6 der Formel

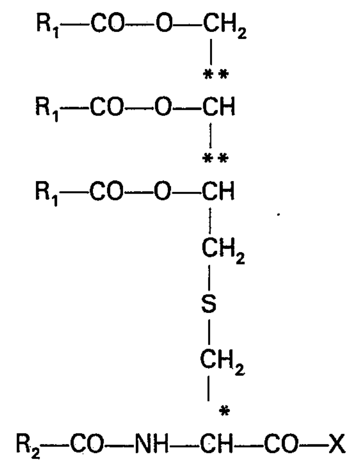

$* = R$

$** = R$ oder S

worin $R_1$ und $R_2$ die in einem der Ansprüche 1—6 gegebene Bedeutung haben, und X eine Aminosäure oder eine Peptidkette mit 2—5 Aminosäuren bedeutet, und Gemische der and den **C-Atomen stereoisomeren Verbindungen, und ihre Salze und Komplexe.

13. Die Amide und Ester der Verbindungen gemäss den Ansprüchen 7—12, worin sich ein Esterrest in der tereminalen veresterten Carboxylgruppe von X von einem niederen aliphatischen Alkohol mit 1—7 C-Atomen, einem Aethylenglykol oder dem Glyzerin ableitet, und eine terminale Amidgruppe in X die Amidgruppe $CONH_2$ ist oder eine sich von einem niederen aliphatischen Amin 1—7 C-Atomen, dem Pyrrolidin, Piperidin oder Piperazin ableitende Amidgruppe ist.

14. Das Lipopeptid N-Palmitoyl-S-[2(R),3-dipalmitoyloxypropyl]-Cys-Ser-Ser-Asn-Ala-Lys-OH, gemäss Anspruch 1.

15. Das Lipopeptid N-Palmitoyl-S-[2(R),3-dipalmitoyloxypropyl]-Cys-Ser-Ser-Phe-Ala-Glu-OH gemäss Anspruch 1.

16. Das N-Palmitoyl-S-[2(R),3-di-(dihydrosterkuloyloxypropyl]-Cys-Ser-Ser-Asn-Ala-Glu-OH gemäss Anspruch 1.

17. Das Lipopeptid N-Lauroyl-S-[2(R),3-dipalmitoyloxypropyl]-Cys-Ser-Ser-Asn-Ala-Glu-OH gemäss Anspruch 1.

18. Das N-Myristoyl-S-[2(R),3-Dipalmitoyloxypropyl]-Cys-Glu-Gin-Asn-Ala-Lys-OH gemäss Anspruch 1.

19. Das N-Palmitoyl-S-[2(R,S),3-dilauroyloxypropyl]-Cys-Phe-Phe-Asn-Ala-Lys-OH gemäss Anspruch 1.

20. Das N-Palmitoyl-S-[2(R),3-dilauroyloxy]-Cys-Ser-Ser-Asn-Ala-Glu-OH gemäss Anspruch 1.

21. Das N-Palmitoyl-S-[3(R),3-dipalmitoyloxypropyl]-Cys-Ala-Ile-Gly-Val-Gly-Ala-Pro-$NH_2$ gemäss Anspruch 1.

22. Das N-Palmitoyl-S-[2(R),3-dioleoyloxypropyl]-Cys-Ser-Ser-Asn-Ala-Glu-OH gemäss Anspruch 1.

23. Das N-Palmitoyl-S-[2(R,S),3-dipalmitoyloxypropyl]-Cys-Phe-Phe-Asn-Ala-Lys-OH gemäss Anspruch 1.

24. Das N-Stearoyl-S-[2(R),3-dipalmitoyloxypropyl]-Cys-Ser-Ser-Asn-Ala-Glu-Ile-Asp-Glu-OH gemäss Anspruch 1.

25. Die Verbindungen der Ansprüche 14—15 entsprechenden Lipopeptide, in denen anstelle der Palmitoylreste im Diacyloxypropylrest Lauroyl-, Stearoyl, Oleoyl-, Behenoyl- oder Dihydrosterkuloyl-Reste vorhanden sind.

26. Eine Verbindung der Ansprüche 14—18, 21 und 23—24 mit R-Konfiguration am 2-C-Atom des Propylrestes entsprechende epimere Verbindung mit S-Konfiguration oder ein Diastereomerengemisch von R- und S-Verbindung.

27. Ammoniumsalze, Alkali- oder Erdalkalisalze von sauren Verbindungen, und pharmazeutisch anwendbare, nichttoxische Säureadditionssalze von basischen Verbindungen, und Kupfer-, Zink-, Eisen- oder Kobalt-Komplexsalze von Verbindungen gemäss einem der Ansprüche 1—26.

28. Verbindungen der Formel

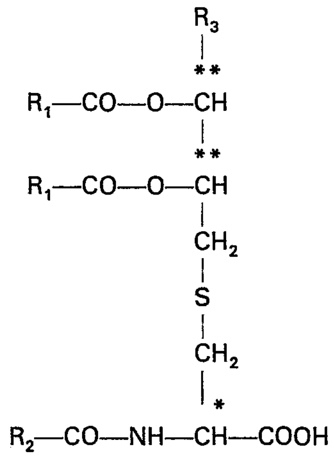

* = R

** = R, S oder Gemisch (R,S)

worin $R_1$, $R_2$ je einen gesättigten oder ungesättigten, aliphatischen oder gemischt aliphatischcycloaliphatischen, gegebenenfalls auch durch Sauerstofffunktionen substituierten Kohlenwasserstoffrest mit 11—21 C-Atomen und $R_3$ Wasserstoff oder den Rest $R_1$—CO—$OCH_2$—, wo $R_1$ die gleiche Bedeutung hat, bedeuten, wobei die mit * bzw. ** bezeichneten Asymmetriezentren die absolute R bzw. S- oder R-Konfiguration besitzen, und Gemische der an den C**-Atomen stereoisomeren Verbindungen.

29. Eine Verbindung gemäss Anspruch 28, worin $R_3$ Wasserstoff bedeutet und worin sich die Acylreste $R_1$ und $R_2$ von der Palmitin-, Stearin-, Oel-, laurin-, Myristin-, Behen-, Dihydrosterkul-, Malval-Hydnocarpus- oder Chaulmoograsäure ableiten.

30. Pharmazeutische Präparate enthaltend eine Verbindung gemäss einem der Ansprüche 1—28.

31. Eine Verbindung gemäss einem der Ansprüche 1—28 zur Anwendung in einem Verfahren für die therapeutische Behandlung des tierischen oder menschlichen Körpers.

32. Verbindungen gemäss einem der Ansprüche 1—20 zur Anwendung als immunstimmulierende Mittel, insbesondere als Adjuvantien bei der Herstellung von Antiseren für Therapie und Diagnostik und bei der Induktion von immunologisch aktivierten Lymphocyten-populationen für Zelltransferverfahren, oder als Prophylaktikum oder Therapeutikum gegen Infektionskrankheiten bei Mensch und Tier.

33. Verfahren zur Herstellung von Verbindungen der Formel

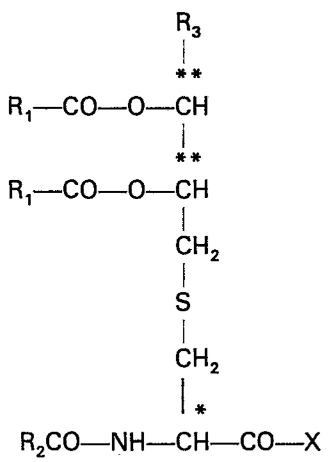

(I)

* = R

** = R oder S

worin $R_1$ und $R_2$ je einen gesättigten oder ungesättigten, aliphatischen oder gemischt aliphatischcycloaliphatischen, gegebenenfalls auch durch Sauerstoffunktionen substituierten, Kohlenwasserstoffrest mit 11—21 C-Atomen, $R_3$ Wasserstoff oder den Rest $R_1$—CO—O—$CH_2$—, wo $R_1$die gleich Bedeutung hat, und X eine peptidisch gebundene natürliche aliphatische Aminosäure mit freier, veresterter

oder amidierter Carboxylgruppe, oder eine Aminosäurensequenz von 2—10 natürlichen aliphatischen Aminosäuren, deren terminale Carboxylgruppe in freier, veresterter oder amidierter Form vorliegt, bedeuten, wobei die mit * bzw. ** bezeichneten Asymmetriezentren die absolute R- bzw. S- oder R-konfiguration besitzen, und gegebenenfalls von Gemischen der an den CII-Atomen epimeren R- und S-Verbindungen, und ihren Salzen und Komplexen, dadurch gekennzeichnet, dass man

a) in einer Verbindung der Formel

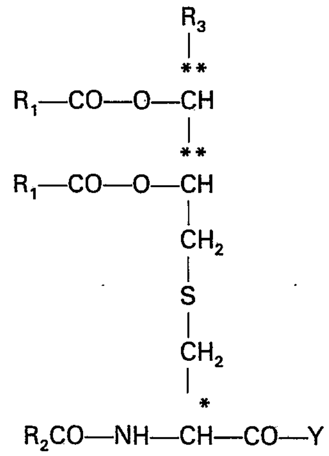

(VI)

* = R

** = R, S oder Gemisch (R,S)

worin $R_1$, $R_2$ und $R_3$ die für Formel (I) angegebene Bedeutung haben und Y eine X in der Formel (I) entsprechende Aminosäure oder Aminosäurensequenz darstellt, worin jedoch mindestens eine der die Aminosäuren substituierenden hydrophilen Gruppen und/oder die terminale Carboxylgruppe durch eine unter neutralen oder milden sauren Bedingungen abspaltbare Schutzgruppe geschützt ist, oder in einem Salz einer solchen Verbindung, die Schutzgruppe(n) abspaltet, oder

b) dass man eine Verbindung der Formel

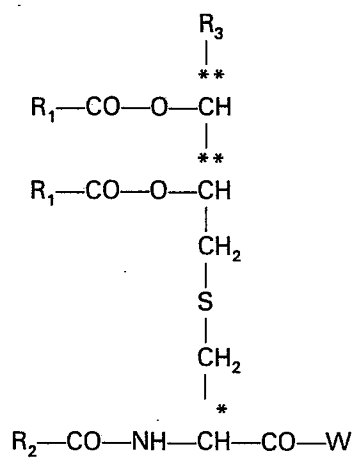

(VII)

* = R

** = R, S oder Gemisch (R,S)

worin $R_1$, $R_2$ und $R_3$ die für Formel (I) angegebene Bedeutung haben und W = OH oder eine Aminosäure oder eine unvollständige Sequenz von Aminosäuren gemäss X in Formel (I) darstellt, mit einer Verbindung der Formel

$$NH_2—Z_1 \quad \text{(VIII)}$$

wo $Z_1$ eine der Gruppe X in Formel (I) entsprechende Gruppe bzw. eine zur genannten Aminosäure oder unvollständigen Sequenz von Aminosäuren gemäss X komplementären Aminosäuresequenz bzw.

Aminosäure bedeutet, in welchen Sequenzen jedoch keine freien Aminogruppen vorhanden sind, oder einem Salz derselben, umsetzt, oder
c) eine Verbindung der Formel

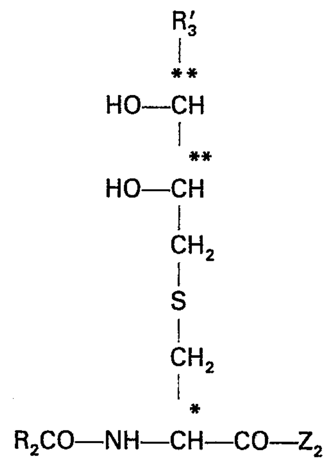

(IX)

* = R

** = R, S oder Gemisch (R,S)

worin $R_2$ die für Formel (I) angegebene Bedeutung besitzt und $R'_3$ Wasserstoff oder die Gruppe $H—O—CH_2—$ darstellt, und $Z_2$ eine X in Formel (I) entsprechende Gruppe darstellt, worin jedoch keine freien Hydroxygruppen vorhanden sind, oder ein Salz dieser Verbindung, in an sich bekannter Weise acyliert, und, wenn erwünscht, in erhaltenen Verbindungen mit freier terminaler Carboxylgruppe, dieselbe in eine Amidgruppe oder Estergruppe überführt, und/oder die Verbindungen in ihre Salze oder Komplexe überführt.

**Revendications**

1. Composé de formule

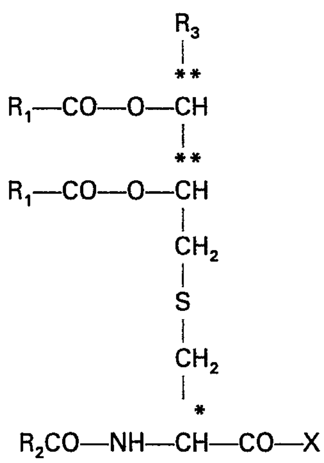

(I)

* = R

** = R ou S

où $R_1$ et $R_2$ représentent chacun un radical hydrocarburé aliphatique ou mixte aliphatique-cycloaliphatique saturé ou insaturé, éventuellement également substitué par des fonctions oxygénées et ayant de 11 à 21 atomes de carbone, $R_3$ un hydrogène ou le radical $R_1—CO—O—CH_2—$ où $R_1$ a la même signification, et X un acide aminé aliphatique naturel à liaison peptidique avec groupe carboxyle libre, estérifié ou amidé, ou une séquence d'acides aminés de 2 à 10 acides aminés aliphatiques naturels, dont le groupe carboxyle terminal se présente sous forme libre, estérifiée ou amidée, où les centres d'asymétrie désignés par * ou ** possèdent la configuration absolute R ou S our R, et éventuellement

les mélanges des composés R et S épimères sur les atomes de carbone **, et les sels et complexes de ces composés.

2. Composés selon la revendication 1, où les radicaux acyle $R_1CO$ et $R_2CO$ dérivent d'acides gras saturés ou insaturés ayant de 14 à 18 atomes de carbone, et où $R_1$ et $R_2$ peuvent être identiques ou différents.

3. Composés selon la revendication 1, où les radicaux acyle dérivent de l'acide palmitique, de l'acide stéarique, de l'acide oléique, de l'acide laurique, de l'acide myristique, de l'acide béhénique, de l'acide dihydrosterculique, de l'acide malvalique, de l'acide hydnocarpique ou de l'acide chaulmoogrique.

4. Composés selon l'une des revendications 1—3, où les acides aminés portant des groupes hydrophiles dans X sont les acides aminés ioniques asparagine, acide glutamique et/ou oxyglutamique, ou la lysine, l'ornithine, l'arginine et/ou l'histidine, ou l'asparagine, la glutamine, la sérine et/ou la thréonine, et où les acides aminés ne portant pas de groupes hydrophiles dans X sont la glycine, l'alanine, la valine, la norvaline, la leucine, l'isoleucine et/ou la méthionine.

5. Composés selon l'une des revendications 1—4, où dans la chaîne peptidique X les acides aminés à caractère hydrophile sont directement liés les uns aux autres.

6. Composés selon la revendication 5, où la séquence des acides aminés à caractère hydrophile est directement liée au groupe carboxyle de la cystéine dans la fraction triacyl-glycéryl- ou tétracyl-, érythrityl-cystéine.

7. Composés selon l'une des revendications 1—6, où X dans la formule 1 représente un acide aminé ou une chaîne peptidique avec de 2 à 5 acides aminés.

8. Composés selon la revendication 1 de formule

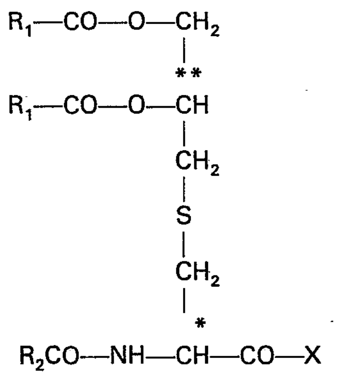

(IV)

* = R
** = R

ou leurs sels et complexes, où X représente un acide aminé aliphatique naturel à liaison peptidique avec un groupe hydrophile et un groupe carboxyle libre, estérifié ou amidé, ou une séquence d'acides aminés de 2 à 5 acides aminés aliphatiques naturels, dont la moitié porte au moins un groupe hydrophile, dont le groupe carboxyle terminal se présente sous forme libre, estérifiée ou amidée, où les centres d'asymétrie représentés par * et ** ont la configuration R.

9. Composés selon l'une des revendications 1—6, où X dans la formule I de la revendication 1 présente une séquence d'acides aminés synthétique par comparaison aux lipopeptides de dégradation de lipoprotéines muréiques connus.

10. Composés correspondants aux composés de le revendication 8, où cependant la configuration sur l'atome de carbone C** est S au lieu de R, et les mélanges des diastéréoisomères R et S.

11. Composés selon l'une des revendications 1—6 de formule

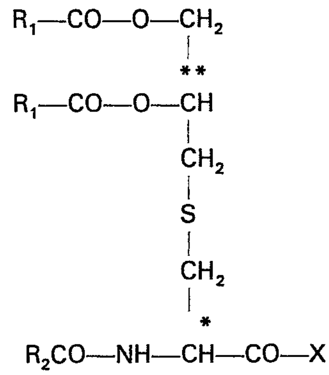

* = R

** = R

où $R_1$ et $R_2$ ont la signification donnée pour la formule I, et où X représente une chaîne peptidique avec de 6 à 10 acides aminés, où les centres d'asymétrie désignés par * et ** ont la configuration R, ainsi que les composés présentant sur l'atome de C** la configuration (S), ainsi que les mélanges de diastéréoisomères R et S, leurs sels et complexes.

12. Composés selon l'une des revendications 1—6 de formule

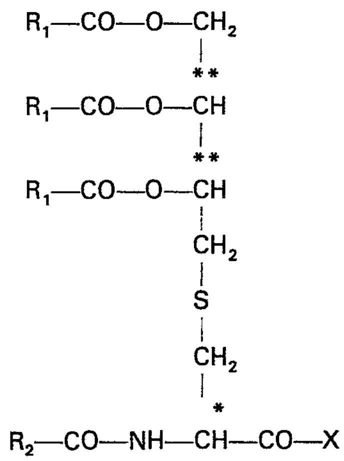

* = R

** = R ou S

où $R_1$ et $R_2$ ont la signification donnée dans l'une des revendications 1—6, et où X représente un acide aminé ou une chaîne peptidique avec de 2 à 5 acides aminés, et les mélanges des composés stéréoisomériques sur les atomes de C**, et leurs sels et complexes.

13. Les amides et esters des composés selon les revendications 7—12, où un radical ester dans le groupe carboxyle estérifié terminal de X dérive d'un alcool aliphatique inférieur avec de 1 à 7 atomes de carbone, d'un éthylèneglycol ou de la glycérine, et où un groupe amide terminal dans X est le groupe amide $CONH_2$ ou bien est un groupe amide dérivant d'une amine aliphatique inférieure ayant de 1 à 7 atomes de carbone, de la pyrrolidine, de la pipéridine ou de la pipérazine.

14. Le lipopeptide N-palmitoyl-S-[2(R),3-dipalmitoyloxypropyl]-Cys-Ser-Ser-Asn-Ala-Lys-OH, selon la revendication 1.

15. Le lipopeptide N-palmitoyl-S-[2(R),3-dipalmitoyloxypropyl]-Cys-Ser-Ser-Phe-Ala-Glu-OH selon la revendication 1.

16. Le N-palmitoyl-S-[2(R),3-di-(dihydrosterculoyloxypropyl]-Cys-Ser-Ser-Asn-Ala-Glu-OH selon la revendication 1.

17. Le lipopeptide N-lauroyl-S-[2(R),3-dipalmitoyloxypropyl]-Cys-Ser-Ser-Asn-Ala-Glu-OH selon la revendication 1.

18. Le N-myristoyl-S-[2(R),3-dipalmitoyloxypropyl]-Cys-Glu-Gln-Asn-Ala-Lys-OH selon la revendication 1.

19. Le N-palmitoyl-S-[2(R,S),3-dilauroyloxypropyl]-Cys-Phe-Phe-Asn-Ala-Lys-OH selon la revendication 1.

20. Le N-palmitoyl-S-[2(R),3-dilauroyloxy]-Cys-Ser-Ser-Asn-Ala-Glu-OH selon la revendication 1.

21. Le N-palmitoyl-S-[3(R),3-dipalmitoyloxypropyl]-Cys-Ala-Ile-Gly-Val-Gly-Ala-Pro-$NH_2$ selon la revendication 1.

22. Le N-palmitoyl-S-[2(R),3-dioléoyloxypropyl]-Cys-Ser-Ser-Asn-Ala-Glu-OH selon la revendication 1.

23. Le N-palmitoyl-S-[2(R,S),3-dipalmitoyloxypropyl]-Cys-Phe-Phe-Asn-Ala-Lys-OH selon la revendication 1.

24. Le N-stéaroyl-S-[2(R),3-dipalmitoyloxypropyl]-Cys-Ser-Ser-Asn-Ala-Glu-Ile-Asp-Glu-OH selon la revendication 1.

25. Les composés des lipopeptides correspondants aux revendications 14—15, où on trouve au lieu des radicaux palmitoyle dans le radical diacyloxypropyle les radicaux lauroyle, stéaroyle, oléoyle, béhénoyle ou dihydrosterculoyle.

26. Composé des revendications 14—18, 21 et 23—24 avec configuration R sur l'atome 2C du composé épimère correspondant au radical propyle avec configuration S ou un mélange de diastéréoisomères des composés R et S.

27. Sels d'ammonium, sels alcalins ou alcalino-terreux de composés acides, et les sels d'addition acides non toxiques pharmaceutiquement acceptables de composés basiques et les sels de complexes de cuivre, de zinc, de fer ou de cobalt de composés selon l'une des revendications 1—26.

28. Composés de formule

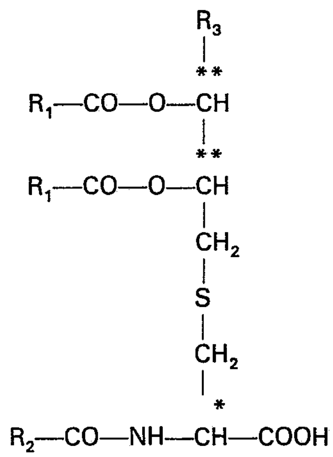

* = R

** = R, S ou mélange (R,S)

où $R_1$, $R_2$ représentent chacun un radical hydrocarbure saturé ou insaturé, aliphatique ou mixte aliphatique-cycloaliphatique, éventuellement également substitué par des fonctions oxygène, et ayant de 11 à 21 atomes de carbone, et $R_3$ un hydrogène ou le radical $R_1$—CO—$OCH_2$—, où $R_1$ a la même signification, où les centres d'asymétrie représentés par * ou ** ont la configuration absolue R ou S, et les mélanges des composés stéréoisomériques sur les atomes de C**.

29. Composé selon la revendication 28, où $R_3$ représente un hydrogène, et où les radicaux acyle $R_1$ et $R_2$ dérivent de l'acide palmitique, de l'acide stéarique, de l'acide oléique, de l'acide laurique, de l'acide myristique, de l'acide béhénique, de l'acide dihydrosterculite, de l'acide malvalique, de l'acide hydnocarpique ou de l'acide chaulmoogrique.

30. Préparations pharmaceutiques contenant un composé selon l'une des revendications 1—28.

31. Composé selon l'une des revendications 1—28 aux fins d'application dans un procédé de traitement thérapeutique du corps animal ou humain.

32. Composés selon l'une des revendications 1—28 aux fins d'application comme agents immunostimulants, en particulier comme adjuvants dans la préparation d'antiséra pour la thérapeutique et le diagnostic et dans l'induction des populations lymphocitaires immunologiquement activées, pour le procédé de transfert cellulaire, ou comme agents prophylactiques ou thérapeutiques contre les maladies infectieuses chez l'homme et l'animal.

33. Procédé de préparation de composés de formule

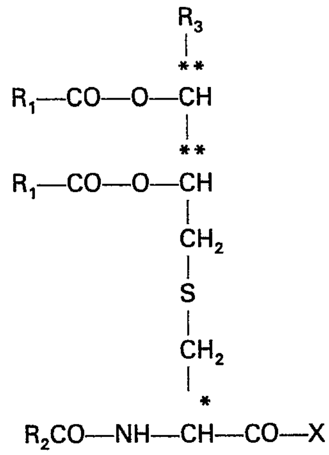

(I)

$* = R$

$** = R$ ou S

où $R_1$ et $R_2$ représentent chacun un radical hydrocarburé aliphatique ou mixte aliphatique-cycloaliphatique saturé ou insaturé, éventuellement également substitué par des fonctions oxygénées et ayant de 11 à 21 atomes de carbone, $R_3$ un hydrogène ou le radical $R_1$—CO—O—$CH_2$— où $R_1$ a la même signification, et X un acide aminé aliphatique naturel à liaison peptidique avec groupe carboxyle libre, estérifié ou amidé, ou une séquence d'acides aminés de 2 à 10 acides aminés aliphatiques naturels, dont le groupe carboxyle terminal se présente sous forme libre, estérifiée ou amidée, où les centres d'asymétrie désignés par * ou ** possèdent la configuration absolue R ou S ou R, et éventuellement les mélanges des composés R et S épimères sur les atomes de carbone **, et les sels et complexes de ces composés, caractérisé en ce que

a) dans un composé de formule

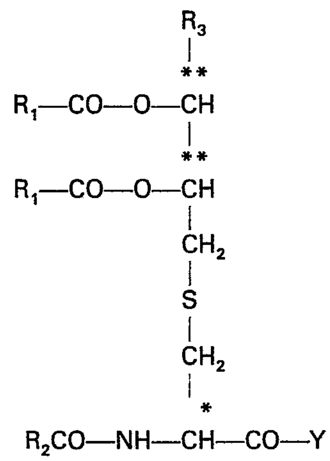

(VI)

$* = R$

$** = R$, S ou mélange (R,S)

où $R_1$, $R_2$ et $R_3$ ont la signification donnée pour la formule (I) et où Y représente un acide aminé ou une séquence d'acides aminés correspondant à X dans la formule (I), où cependant au moins l'un des groupes hydrophiles substituant les acides aminés et/ou le groupe carboxyle terminal est protégé par un groupe protecteur séparable dans des conditions neutres ou faiblement acides, ou dans un sel d'un tel composé, on sépare le ou les groupes protecteurs ou

b) on fait réagir un composé de formule

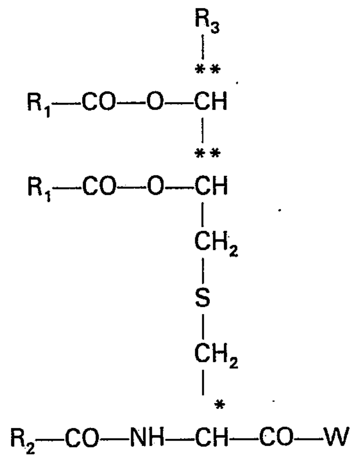

(VII)

* = R

** = R, S ou mélange (R,S)

où $R_1$, $R_2$ et $R_3$ ont la signification donnée pour la formule (I) et où W = OH ou un acide aminé ou une séquence incomplète d'acides aminés selon X dans la formule (I), avec un composé de formule

$$NH_2—Z_1 \qquad (VIII)$$

où $Z_1$ représente un groupe correspondant au groupe X dans la formule (I) ou une séquence d'acides aminés ou un acide aminé complémentaire de l'acide aminé ou de la séquence incomplète d'acides aminés mentionné(e) selon X, dans laquelle séquence on ne trouve cependant aucun groupe amino libre, ou un de ses sels, ou

c) on acyle de façon classique un composé de formule

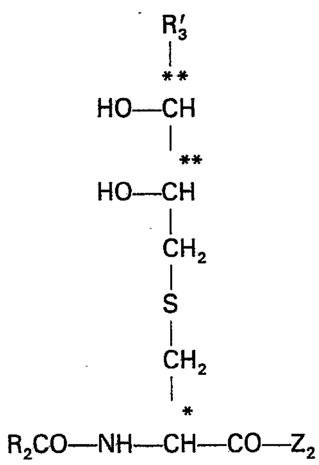

(IX)

* = R

** = R, S ou mélange (R,S)

où $R_2$ a la signification donnée pour la formule (I) et $R_3'$ représente un hydrogène ou le groupe $HO—CH_2$, et $Z_2$ un groupe correspondant à X dans la formule (I), où cependent aucun groupe hydroxy libre n'est présent, ou un sel de ce composé, et, si on le désire, on transforme dans les composés obtenus à groupe carboxyle terminal libre celui-ci en un groupe amide ou en un groupe ester, et/ou les composés en leurs sels ou complexes.

**Claims**

1. Compounds of the formula

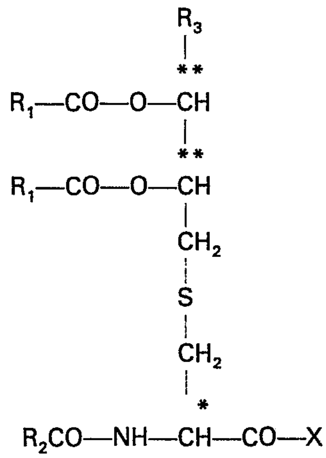

(I)

* = R

** = R or S

wherein $R_1$ and $R_2$ each represent a saturated or unsaturated aliphatic or mixed aliphatic-cycloaliphatic hydrocarbon radical which has 11—21 C atoms and which is also optionally substituted by oxygen functions, $R_3$ represents hydrogen or the radical $R_1$—CO—O—$CH_2$—, where $R_1$ has the same meaning, and X represents a natural aliphatic amino acid linked by a peptide bond and having a free, esterified or amidated carboxyl group, or an amino acid sequence of 2—10 natural aliphatic amino acids, the terminal carboxyl group of which is free or in the ester or amide form, with the asymmetric centres denoted by * and ** possessing the absolute R- and S- or R-configurations, respectively; and, possibly, mixtures of the R- and S-compounds epimeric on the C** atoms, and salts and complexes of all these compounds.

2. Compounds according to Claim 1, wherein the acyl groups $R_1CO$ and $R_2CO$ are derived from saturated or unsaturated fatty acids having 14—18 C atoms, and wherein $R_1$ and $R_2$ can be identical or different.

3. Compounds according to Claim 1, wherein the acyl groups are derived from palmitic, stearic, oleic, lauric, myristic, behenic, dihydrosterculic, malvalic, hydnocarpic or chaulmoogric acid.

4. Compounds according to any one of Claims 1—3, wherein amino acids carrying hydrophilic groups in X are the ionic amino acids: aspartic acid, glutamic acid, and/or oxyglutamic acid, or: lysine, ornithine, arginine and/or histidine, or asparagine, glutamine, serine and/or threonine, and amino acids carrying no hydrophilic groups in X are glycine, alanine, valine, norvaline, leucine, isoleucine and/or methionine.

5. Compounds according to any one of Claims 1—4, wherein in the peptide chain X the amino acids having hydrophilic character are linked directly with each other.

6. Compounds according to Claim 5, wherein the sequence of the amino acids having hydrophilic character is bound directly to the carboxyl group of the cysteine in the triacyl-glycerylcysteine part or tetraacyl-erythritylcysteine part.

7. Compounds according to any one of Claims 1—6, wherein X in the formula I represents an amino acid, or a peptide chain having 2—5 amino acids.

8. Compounds according to Claim 1 of the formula

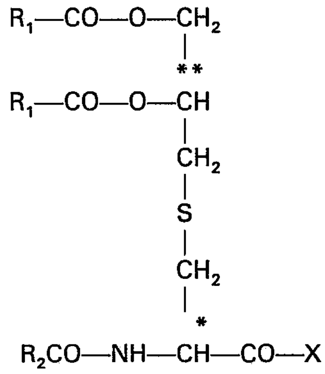

(IV)

* = R

** = R

their salts and complexes, wherein X represents a natural aliphatic amino acid linked by a peptide bond and having a hydrophilic group and a free, esterified or amidated carboxyl group, or an amino acid sequence of 2—5 natural aliphatic amino acids of which half carry at least one hydrophilic group, and of which the terminal carboxyl group is free or in the ester or amide form, with the asymmetic centres denoted by * and ** possessing the R-configuration.

9. Compounds according to any one of Claims 1—6, wherein X in formula I of Claim 1 represents an unnatural amino acid sequence compared with that of the known murein-lipoprotein-degradation lipopeptides.

10. Compounds corresponding to those of Claim 8, in which however the configuration on the C** atom is S instead of R, and the mixtures of the R- and S-diastereomers.

11. Compounds according to any one of Claims 1—6 of the formula

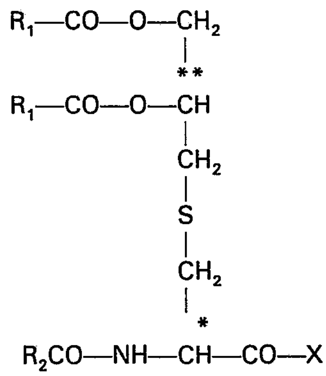

* = R

** = R

wherein $R_1$ and $R_2$ have the meanings given under the formula I, and X represents a peptide chain having 6—10 amino acids, with the asymmetric centres denoted by * and ** possessing the R-configuration, and also the compounds having on the C** atom the (S)-configuration, as well as R- and S-diastereomers mixtures, and salts and complexes thereof.

12. Compounds according to any one of Claims 1—6 of the formula

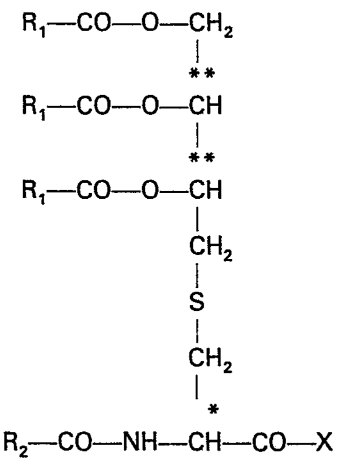

* = R

** = R or S

wherein $R_1$ and $R_2$ have the meanings given in any one of Claims 1—6, and X represents an amino acid, or a peptide chain having 2—5 amino acids, and mixtures of the compounds stereoisomeric on the C** atoms, and salts and complexes thereof.

13. The amides and esters of the compounds according to Claims 7—12, wherein an ester radical in the terminally esterified carboxyl group of X is derived from a lower aliphatic alcohol having 1—7 C atoms, an ethylene glycol or glycerin, and a terminal amide group in X is the amide group $CONH_2$, or an amide group derived from a lower aliphatic amine having 1—7 C atoms, or from pyrrolidine, piperidine or piperazine.

14. The lipopeptide: N-palmitoyl-S-[2(R),3-dipalmitoyloxypropyl]-Cys-Ser-Ser-Asn-Ala-Lys-OH, according to Claim 1.

15. The lipopeptide: N-palmitoyl-S-[2(R),3-dipalmitoyloxypropyl]-Cys-Ser-Ser-Phe-Ala-Glu-OH, according to Claim 1.

16. N - palmitoyl - S - [2(R),3 - di - (dihydrosterculoyloxypropyl] - Cys - Ser - Ser - Asn - Ala - Glu - OH, according to Claim 1.

17. The lipopeptide: N-lauroyl-S-[2(R),3-dipalmitoyloxypropyl]-Cys-Ser-Ser-Asn-Ala-Glu-OH, according to Claim 1.

18. N-Myristoyl-S-[2-(R),3-dipalmitoyloxypropyl]-Cys-Glu-Gln-Asn-Ala-Lys-OH, according to Claim 1.

19. N-Palmitoyl-S-[2(R,S),3-dilauroyloxypropyl]-Cys-Phe-Phe-Asn-Ala-Lys-OH, according to Claim 1.

20. N-palmitoyl-S-[2(R),3-dilauroyloxy]-Cys-Ser-Ser-Asn-Ala-Glu-OH, according to Claim 1.

21. N-palmitoyl-S-[3(R),3-dipalmitoyloxypropyl]-Cys-Ala-Ile-Gly-Val-Gly-Ala-Pro-$NH_2$, according to Claim 1.

22. N-palmitoyl-S-[2(R),3-dioleoyloxypropyl]-Cys-Ser-Ser-Asn-Ala-Glu-OH, according to Claim 1.

23. N-Palmitoyl-S-[2(R,S),3-dipalmitoyloxypropyl]-Cys-Phe-Phe-Asn-Ala-Lys-OH, according to Claim 1.

24. N - Stearoyl - S - [2(R),3 - dipalmitoyloxypropyl] - Cys - Ser - Ser - Asn - Ala - Glu - Ile - Asp - Glu - OH, according to Claim 1.

25. The lipopeptides corresponding to the compounds of Claims 14—15, in which instead of palmitoyl radicals being present in the diacyloxypropyl radical, there are present lauroyl, stearoyl, oleoyl, behenoyl or dihydrosterculoyl radicals.

26. A compound of Claims 14—18, 21 and 23—24 having the R-configuration on the 2-C atom of the propyl group, a corresponding epimeric compound having the S-configuration, or R- and S-diastereomers mixtures.

27. Ammonium salts, alkali metal or alkaline-earth metal salts of acid compounds, and pharmaceutically applicable nontoxic acid addition salts of basic compounds, and copper-, zinc-, iron- or cobalt-complex salts of compounds according to any one of Claims 1—26.

28. Compound of the formula

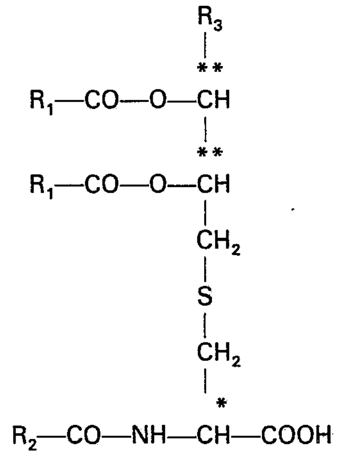

* = R

** = R, S or mixture (R,S)

wherein $R_1$ and $R_2$ each represent a saturated or unsaturated, aliphatic or mixed aliphatic-cycloaliphatic hydrocarbon radical which has 11—21 C atoms and which is also optionally substituted by oxygen functions, and $R_3$ represents hydrogen or the radical $R_1$—CO—$OCH_2$—, where $R_1$ has the same meaning, with the asymmetric centres denoted by * and ** having the absolute R- and S- or R-configurations, respectively, and mixtures of the compounds stereoisomeric on the C** atoms.

29. A compound according to Claim 28, wherein $R_3$ represents hydrogen, and wherein the acyl groups $R_1$ and $R_2$ are derived from palmitic, stearic, oleic, lauric, myristic, behenic, dihydrosterculic, malvalic, hydnocarpic or chaulmoogric acid.

30. Pharmaceutical preparations containing a compound according to any one of Claims 1—28.

31. A compound according to any one of Claims 1—28 for application in a process for the therapeutic treatment of the animal or human body.

32. Compounds according to any one of Claims 1—28 for application as immunity-stimulating therapeutic preparations, especially as adjuvants in the production of antisera for therapy and diagnosis, and in the induction of immunologically activated lymphocyte populations for cell-transfer processes, or as prophylactics or therapeutics against infectious diseases in humans and animals.

33. A process for producing compounds of the formula

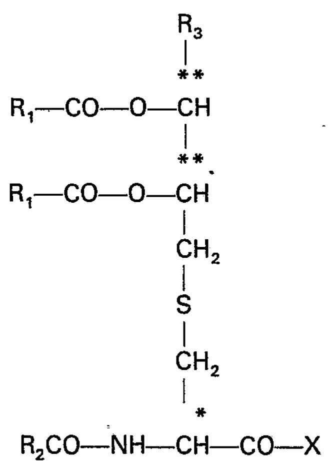

(I)

* = R

** = R or S

wherein $R_1$ and $R_2$ each represent a saturated or unsaturated aliphatic or mixed aliphatic-cycloaliphatic hydrocarbon radical which has 11—21 C atoms and which is also optionally substituted by oxygen functions, $R_3$ represents hydrogen or the radical $R_1$—CO—O—$CH_2$—, where $R_1$ has the same meaning, and X represents a natural aliphatic amino acid linked by a peptide bond and having a free, esterified or amidated carboxyl group, or an amino acid sequence of 2—10 natural aliphatic amino acids, the

terminal carboxyl group of which is free or in the ester or amide form, with the asymmetric centres denoted by * and ** possessing the absolute R- and S- or R-configurations, respectively; and optionally mixtures of the R- and S-compounds epimeric on the C** atoms, and salts and complexes thereof, whereby

a) in a compound of the formula

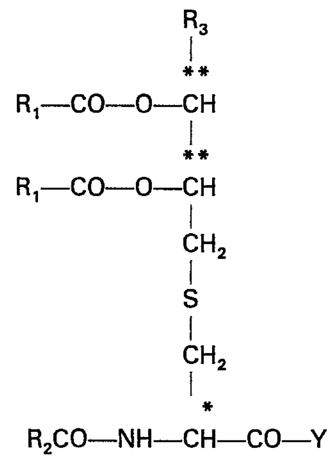

(VI)

* = R

** = R, S or mixture R, S

wherein $R_1$, $R_2$ and $R_3$ have the meanings given under the formula (I) of Claim 1, and Y represents an amino acid or amino acid sequence corresponding to X in the said formula I, wherein however at least one of the hydrophilic groups substituting the amino acids and/or the terminal carboxyl group is (are) protected by a protective group which can be split off under neutral or mild acid conditions, or in a salt of such a compound, the protective groups(s) is or are split off; or

b) a compound of the formula

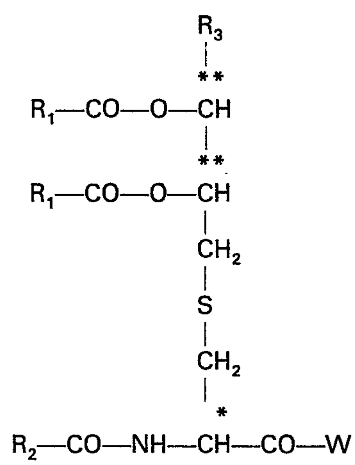

(VII)

* = R

** = R, S or mixture (R,S)

wherein $R_1$, $R_2$ and $R_3$ have the meanings given under the said formula (I), and W represents OH or an amino acid or an incomplete sequence of amino acids according to X in the said formula I, is reacted with a compound of the formula

$$NH_2—Z_1 \qquad (VIII)$$

where $Z_1$ represents a group corresponding to the group X in the said formula (I), or an amino acid sequence or amino acid complementary to the stated amino acid or incomplete sequence of amino acids according to X, in which sequences however no free amino groups are present, or with a salt thereof; or

c) a compound of the formula

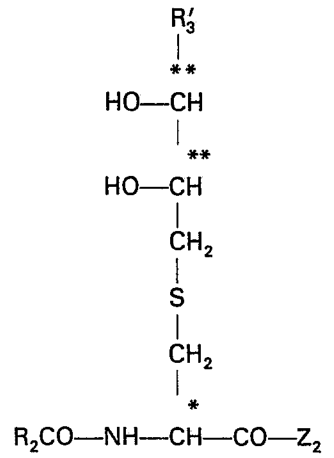

(IX)

* = R

** = R, S or mixture (R,S)

wherein $R_2$ has the meaning given under the said formula (I), and $R_3'$ represents hydrogen or the group H—O—$CH_2$—, and $Z_2$ represents a group corresponding to X in the said formula (I), wherein however no free hydroxyl groups are present, or a salt of this compound, is acylated in a manner known per se; and, if required, in resulting compounds having a free terminal carboxyl group this group is converted into an amide group or ester group, and/or the compounds are converted into salts or complexes thereof.